# EUROPEAN PATENT APPLICATION

(11) **EP 4 397 749 A1**
(43) Date of publication of application: **10.07.2024**
(21) Application number: 22864719.4
(22) Date of filing: 02.09.2022
(51) Int. Cl.: C12N 5/071, A01K 67/027, A61L 27/36, A61L 27/38, A61P 13/12, G01N 33/50

(54) **KIDNEY ORGANOID AND METHOD FOR PRODUCING SAME**

(30) Priority: 03.09.2021 JP 2021144199
(71) Applicant: Otsuka Pharmaceutical Co., Ltd., Tokyo 101-8535 (JP); RIKEN, Wako-shi, Saitama 351-0198 (JP)
(72) Inventor: TAKASATO, Minoru, Wako-shi, Saitama 351-0198 (JP); SAHARA, Yoshiki, Osaka-shi, Osaka 540-0021 (JP)
(74) Representative: Hoffmann Eitle
(86) International application number: PCT/JP2022/033078
(87) International publication number: WO 2023/033137

(57) **Abstract**

An object of the present invention is to a new method of producing a kidney organoid. The method for producing a kidney organoid is characterized by comprising culturing an early kidney organoid with a medium containing an RXR agonist and a PPAR agonist, wherein the kidney organoid includes matured proximal tubule cells.

## Description

### TECHNICAL FIELD

The present invention relates to a kidney organoid and a method for producing the same. More specifically, the present invention relates to a kidney organoid, including matured proximal tubular cells, and a method for producing the same. The present invention also relates to a regenerative medicine composition, including a kidney organoid containing matured proximal tubular cells. The present invention also relates to a non-human mammal, including a kidney organoid containing matured proximal tubular cells and a method for producing the same. The present invention also relates to a method for evaluating drug responsiveness to a test substance. The invention also relates to a method for promoting the maturation of proximal tubule.

### BACKGROUND ART

The kidney concentrates metabolites (for example, urea, uric acid, and creatinine), produced by cells in the body through biological activity, into urine and excretes them from the body. The kidney also regulates the amount and physicochemical properties (for example, electrolyte concentration, osmotic pressure, pH) of extracellular fluid, which is the substantial survival environment for cells in the body.

The kidney is a collection of nephrons including, as functional units, kidney corpuscles (glomerulus and Bowman's capsule) and kidney tubules. Blood filtered by the glomerulus (original urine) passes through the kidney tubules and becomes urine. The kidney tubule starts from the proximal tubule following the glomerulus, and then is connected, through the intermediate tubule, the distal tubule, and the connecting tubule, to the cortical collecting duct.

A method for producing a kidney organoid from human iPS cells has been reported (Patent Literatures 1 and 2).

Non-Patent Literature 1 has reported that Fenofibrate, a peroxisomal proliferation-activated receptor alpha (PPARA) agonist, increases the expression of proximal tubular genes in proximal tubular cells of mice.

### CITATION LIST

Patent Literature 1: JP 2016-527878 A
Patent Literature 2: JP 2017-537655 A

Non-Patent Literature 1: Dhillon P, et. al., Cell Metabolism Volume 33, Issue 2, 2 February 2021, Pages 379-394.e8

### SUMMARY

### TECHNICAL PROBLEM

There is a need in the art for a kidney organoid including matured proximal tubules. There is a need in the art for a method for evaluating drug responsiveness to a test substance by using the kidney organoid possessing an increased susceptibility to kidney damage.

An object of the present disclosure is to provide a novel kidney organoid including matured proximal tubule cells. An object of the present disclosure is to provide a novel method for producing the kidney organoid including matured proximal tubule cells. An object of the present disclosure is to provide a novel regenerative medicine composition, including the kidney organoid containing matured proximal tubule cells. An object of the present disclosure is also to provide a novel method for evaluating drug responsiveness to a test substance. An object of the present disclosure is also to provide a method for promoting the maturation of proximal tubule.

### SOLUTION TO PROBLEM

The present inventors have found that culturing a kidney organoid in the presence of a combination of a PPARA agonist and an RXR agonist results in the formation of the kidney organoid including matured proximal tubule cells. The formed kidney organoid possessed an increased susceptibility to drug responsiveness to cisplatin at low concentrations.

Based on the above findings, the present disclosure provides the inventions according to the following aspects.
[Item 1] A method for producing a kidney organoid, comprising culturing an early kidney organoid with a medium containing an RXR agonist and a PPAR agonist, wherein the kidney organoid includes matured proximal tubule cells.
   [Item 1-1] The method according to item 1, wherein the PPAR agonist is at least one selected from the group consisting of a PPARA agonist, a PPARG agonist, a PPARD agonist, a PPARA/G agonist, a PPARA/D agonist, a PPARG/D agonist, and a PPARA/G/D agonist.
   [Item 1-2] The method according to item 1 or 1-1, wherein the PPAR agonist is a PPARA agonist.
[Item 2] The method according to any one of items 1 to 1-2, further comprising deriving the early kidney organoid from intermediate mesoderm cells, wherein the deriving of the early kidney organoid comprises: culturing the intermediate mesoderm cells with an induction medium B containing a fibroblast growth factor and substantially free of an RXR agonist to form an intermediate mesoderm spheroid; and culturing the intermediate mesoderm spheroid with an induction medium A containing a GSK3β inhibitor, then with the induction medium B containing a fibroblast growth factor and substantially free of an RXR agonist.
   [Item 2-1] The method according to item 2, wherein the induction medium B substantially free of an RXR agonist is further substantially free of a PPAR agonist.
[Item 3] The method according to item 2 or 2-1, further comprising deriving the intermediate mesoderm cells from a pluripotent stem cell, wherein the deriving of the intermediate mesoderm cells comprises: culturing the pluripotent stem cell with an induction medium A containing a GSK3β inhibitor, then with an induction medium B containing a fibroblast growth factor and substantially free of an RXR agonist.
[Item 4] The method according to any one of items 1 to 3, wherein the kidney organoid expresses a proximal tubule marker, wherein the proximal tubule marker is at least one selected from the group consisting of UGT2A3, AZGP1, SLC39A4, BHMT, ACE2, AQP6, CUBN, LRP2, and ABCB1.
   [Item 4-1] The method according to any one of items 1 to 3, wherein the kidney organoid expresses a proximal tubule marker, wherein the proximal tubule marker is at least one selected from the group consisting of UGT2A3, AZGP1, SLC39A4, BHMT, ACE2, AQP6, CUBN, LRP2, ABCB1, SERPINA1, APOA1, and SPARCL1.
[Item 5] The method according to item 4, wherein the kidney organoid further expresses a PPARA downstream gene marker, and the PPARA downstream gene marker is at least one selected from the group consisting of APOA1, APOC3, CPT1A, FABP1, CYP27A1, and ACOX1.
[Item 6] The method according to item 4 or 5, wherein an expression level of the at least one proximal tubule marker in the kidney organoid is 1.2 times or more higher than an expression level of the at least one proximal tubule marker in the early kidney organoid.
   [Item 6-1] The method according to item 5, wherein an expression level of the at least one PPARA downstream gene marker in the kidney organoid is 1.2 times or more higher than an expression level of the at least one PPARA downstream gene marker in the early kidney organoid.
   [Item 6-2] The method according to item 5, wherein an expression level of the at least one proximal tubule marker in the kidney organoid is 1.5 times or more higher than an expression level of the at least one proximal tubule marker in the early kidney organoid, and an expression level of the at least one PPARA downstream gene marker in the kidney organoid is 1.2 times or more higher than an expression level of the at least one PPARA downstream gene marker in the early kidney organoid.
   [Item 6-3] The method according to item 6, wherein the number of the at least one proximal tubule marker whose expression level in the kidney organoid is 1.2 times higher than an expression level in the early kidney organoid is at least one (for example, one, two, three, four, five, or six).
   [Item 6-4] The method according to item 6-1, wherein the number of the at least one PPARA downstream gene marker whose expression level in the kidney organoid is 1.2 times higher than an expression level in the early kidney organoid is at least one (for example, one, two, three, four, five, or six).
   [Item 6-5] The method according to item 6-2, wherein the number of the at least one proximal tubule marker whose expression level in the kidney organoid is 1.2 times higher than an expression level in the early kidney organoid is at least one (for example, one, two, three, four, five, or six), and the number of the at least one PPARA downstream gene marker whose expression level in the kidney organoid is 1.2 times higher than an expression level in the early kidney organoid is at least one (for example, one, two, three, four, five, or six).
   [Item 6-6] The method according to item 4 or 5, wherein the number of the at least one proximal tubule marker whose expression level in the kidney organoid is significantly higher than an expression level in the early kidney organoid is at least one (for example, one, two, three, four, five, or six).
   [Item 6-7] The method according to item 5, wherein the number of the at least one PPARA downstream gene marker whose expression level in the kidney organoid is significantly higher than an expression level in the early kidney organoid is at least one (for example, one, two, three, four, five, or six).
   [Item 6-8] The method according to item 5 or 6-7, wherein the number of the at least one proximal tubule marker whose expression level in the kidney organoid is significantly higher than an expression level in the early kidney organoid is at least one (for example, one, two, three, four, five, or six), and the number of the at least one PPARA downstream gene marker whose expression level in the kidney organoid is significantly higher than an expression level in the early kidney organoid is at least one (for example, one, two, three, four, five, or six).
[Item 7] The method according to any one of items 1 to 6-8, wherein the PPAR agonist is at least one PPARA agonist selected from the group consisting of CP775146, pirinic acid (WY14643), bezafibrate, fenofibrate, gemfibrozil, clofibrate, ciprofibrate, pemafibrate, binifibrate, clinofibrate, clofibric acid, nicofibrate, pirifibrate, plafibride, ronifibrate, theofibrate, tocofibrate, SR10171, GW6471, Fenofibrate, Pirinixic acid, GW7647, Icariin, Eupatilin, Gemfibrozil, Palmitelaidic Acid, NXT629, Saroglitazar Magnesium, Saroglitazar, Oleoylethanolamide, BMS-687453, Gypenoside XLIX, CUDA, Ciprofibrate impurity A, CP-868388 free base, AVE-8134, linoleic acid, linolenic acid, and PPARα-MO-1; at least one PPARG agonist selected from the group consisting of rosiglitazone, troglitazone, pioglitazone, efatutazone, ATx08-001, OMS-405, CHS-131, THR-0921, SER-150-DN, KDT-501, GED-0507-34-Levo, CLC-3001, ALL-4, Rosiglitazone, Rosiglitazone hydrochloride, Troglitazone, T0070907, 5-Aminosalicylic Acid, GW1929, Rosiglitazone maleate, Astaxanthin, Magnolol, Glabridin, Balaglitazone, Mifobate, Inolitazone dihydrochloride, EHP-101, Adelmidrol, Oroxin A, Cefminox sodium, Methyl oleanonate, 15-Deoxy-Δ-12,14-prostaglandin 12, GSK376501A, 4-O-Methyl honokiol, Caulophyllogenin, Darglitazone, Angeloylgomisin H, DS-6930, Inolitazone, and Arhalofenate; at least one PPARD agonist selected from the group consisting of finadelpar, ASP0367, GW501516, Pparδ agonist 5, MBX8025, GW0742, L165041, HPP-593, and NCP-1046; at least one PPARA/G agonist selected from the group consisting of sarogritazar, allegritazar, muragritazar, tesaglitazar, and DSP-8658; a PPARA/D agonist that is one or both of ELA and T913659; a PPARG/D agonist that is one or both of linoleic acid and T3D-959; or at least one PPARA/G/D agonist selected from the group consisting of IVA337, TTA, Bavachinin, GW4148, GW9135, benzafibrate, lobeglitazone, and CS038; or a combination thereof, and/or
   an RXR agonist is at least one selected from the group consisting of 9-cis retinoic acid (alitretinoin), AGN195204, TTNPB (arotinoid acid), Adapalene, Bexarotene, Tazarotene, Tamibarotene, CH55, and AM580.
[Item 8] A kidney organoid including matured proximal tubule cells produced by the method according to any one of items 1 to 7.
[Item 9] A kidney organoid, wherein the kidney organoid includes matured proximal tubule cells, the kidney organoid expresses a proximal tubule marker, and the proximal tubule marker is at least one selected from the group consisting of UGT2A3, AZGP1, SLC39A4, BHMT, ACE2, AQP6, CUBN, LRP2, and ABCB1.
   [Item 9-1] A kidney organoid, wherein the kidney organoid includes matured proximal tubule cells, the kidney organoid expresses a proximal tubule marker, and the proximal tubule marker is at least one selected from the group consisting of UGT2A3, AZGP1, SLC39A4, BHMT, ACE2, AQP6, CUBN, LRP2, ABCB1, SERPINA1, APOA1, and SPARCL1.
[Item 10] The kidney organoid according to item 9, further expressing at least one PPARA downstream gene marker selected from the group consisting of APOA1, APOC3, CPT1A, FABP1, CYP27A1, and ACOX1.
[Item 11] The kidney organoid according to item 9 or 10, wherein an expression level of the at least one proximal tubule marker from the proximal tubule marker is 1.2 times or more higher than an expression level of the at least one proximal tubule marker from the proximal tubule marker in the early kidney organoid.
   [Item 11-1] The kidney organoid according to item 10, wherein an expression level of the at least one PPARA downstream gene marker in the kidney organoid is 1.2 times or more higher than an expression level of the at least one PPARA downstream gene marker in the early kidney organoid.
   [Item 11-2] The kidney organoid according to item 10, wherein an expression level of the at least one proximal tubule marker in the kidney organoid is 1.5 times or more higher than an expression level of the at least one proximal tubule marker in the early kidney organoid, and an expression level of the at least one PPARA downstream gene marker in the kidney organoid is 1.2 times or more higher than an expression level of the at least one PPARA downstream gene marker in the early kidney organoid.
   [Item 11-3] The kidney organoid according to item 11, wherein the number of the at least one proximal tubule marker whose expression level in the kidney organoid is 1.2 times higher than an expression level in the early kidney organoid is at least one (for example, one, two, three, four, five, or six).
   [Item 11-4] The kidney organoid according to item 11-1, wherein the number of the at least one PPARA downstream gene marker whose expression level in the kidney organoid is 1.2 times higher than an expression level in the early kidney organoid is at least one (for example, one, two, three, four, five, or six).
   [Item 11-5] The kidney organoid according to item 11-2, wherein the number of the at least one proximal tubule marker whose expression level in the kidney organoid is 1.2 times higher than an expression level in the early kidney organoid is at least one (for example, one, two, three, four, five, or six), and the number of the at least one PPARA downstream gene marker whose expression level in the kidney organoid is 1.2 times higher than an expression level in the early kidney organoid is at least one (for example, one, two, three, four, five, or six).
   [Item 11-6] The kidney organoid according to item 9 or 10, wherein the number of the at least one proximal tubule marker whose expression level in the kidney organoid is significantly higher than an expression level in the early kidney organoid is at least one (for example, one, two, three, four, five, or six).
   [Item 11-7] The kidney organoid according to item 10, wherein the number of the at least one PPARA downstream gene marker whose expression level in the kidney organoid is significantly higher than an expression level in the early kidney organoid is at least one (for example, one, two, three, four, five, or six).
   [Item 11-8] The kidney organoid according to item 10 or 11-7, wherein the number of the at least one proximal tubule marker whose expression level in the kidney organoid is significantly higher than an expression level in the early kidney organoid is at least one (for example, one, two, three, four, five, or six), and the number of the at least one PPARA downstream gene marker whose expression level in the kidney organoid is significantly higher than an expression level in the early kidney organoid is at least one (for example, one, two, three, four, five, or six).
[Item 12] A method for producing a non-human mammal comprising a kidney organoid in a kidney or a surrounding area thereof, the method comprising introducing a kidney organoid produced by the method according to any one of items 1 to 7, or the kidney organoid according to any one of items 8 to 11-8 into a kidney of a non-human mammal or a surrounding area thereof, wherein the kidney organoid includes matured proximal tubule cells.
[Item 13] A non-human mammal comprising a kidney organoid produced by the method according to any one of items 1 to 7, or the kidney organoid according to any one of items 8 to 11-8 in a kidney or a surrounding area thereof.
[Item 14] A regenerative medicine composition for treating kidney damage or disease, comprising a kidney organoid produced by the method according to any one of items 1 to 7, or the kidney organoid according to any one of items 8 to 11-8.
[Item 15] A method for evaluating drug responsiveness to a test substance, the method comprising: contacting a kidney organoid produced by the method according to any one of items 1 to 7, the kidney organoid according to any one of items 8 to 11-8, a non-human mammal produced by the method according to item 12, or the non-human mammal according to item 13 with the test substance; and measuring drug responsiveness in the kidney organoid or the non-human mammal to the test substance.
   [Item 15-1] The method according to item 15, further comprising evaluating nephrotoxicity of the test substance based on a drug responsiveness measurement result.
[Item 16] A method for treating kidney damage or disease, comprising introducing a kidney organoid produced by the method according to any one of items 1 to 7, the kidney organoid according to any one of items 8 to 11-8, or the medicine composition according to item 14 into a kidney of a mammal that needs treatment or a surrounding area thereof.
[Item 17] A method for promoting proximal tubule maturation, wherein an early kidney organoid is cultured with a medium containing an RXR agonist and a PPAR agonist.

### BRIEF DESCRIPTION OF DRAWINGS

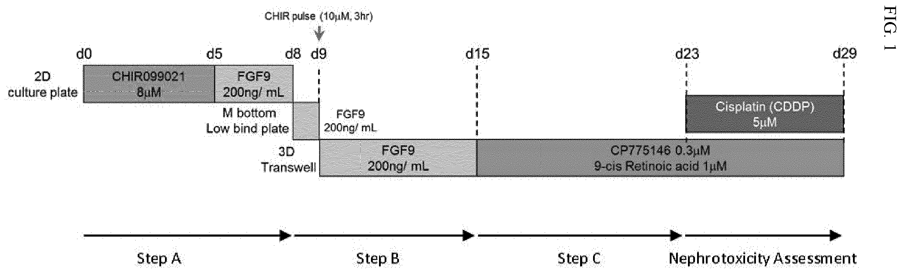

[FIG. 1] Figure 1 is a flowchart outlining the production of a kidney organoid including matured proximal tubule cells according to one embodiment and the nephrotoxicity assessment of drug.

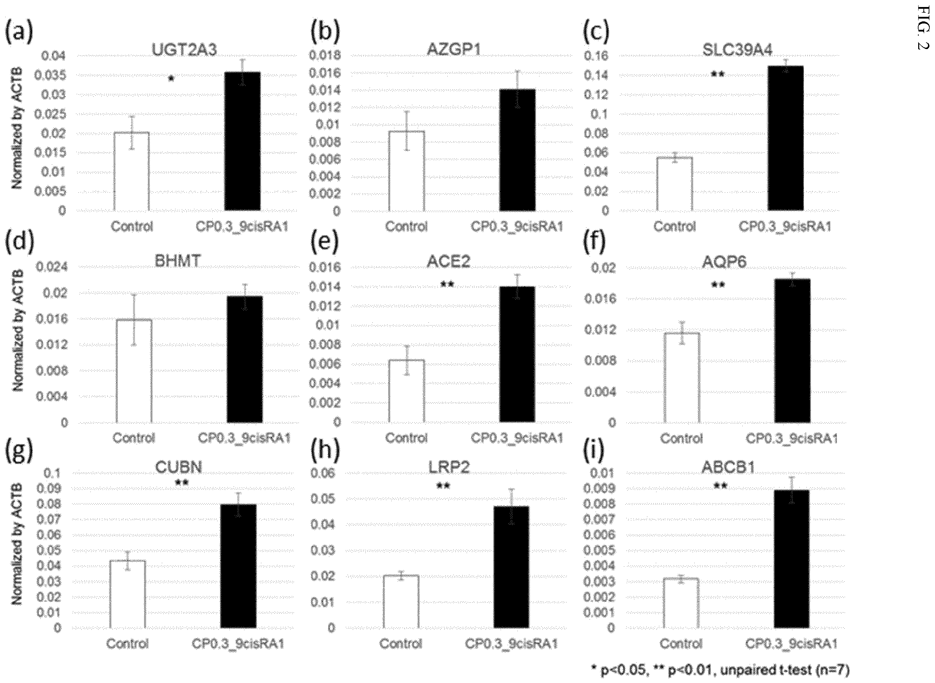

[FIG. 2] Figures 2a to 2i are a series of bar graphs showing expression levels of proximal tubule markers (Figure 2a: UGT2A3, Figure 2b: AZGP1, Figure 2c: SLC39A4, Figure 2d: BHMT, Figure 2e: ACE2, Figure 2f: AQP6, Figure 2g: CUBN, Figure 2h: LRP2, and Figure 2i: ABCB1) in LTL-positive cells derived from kidney organoids cultured in the presence of a combination of CP775146 and 9-cis retinoic acid.

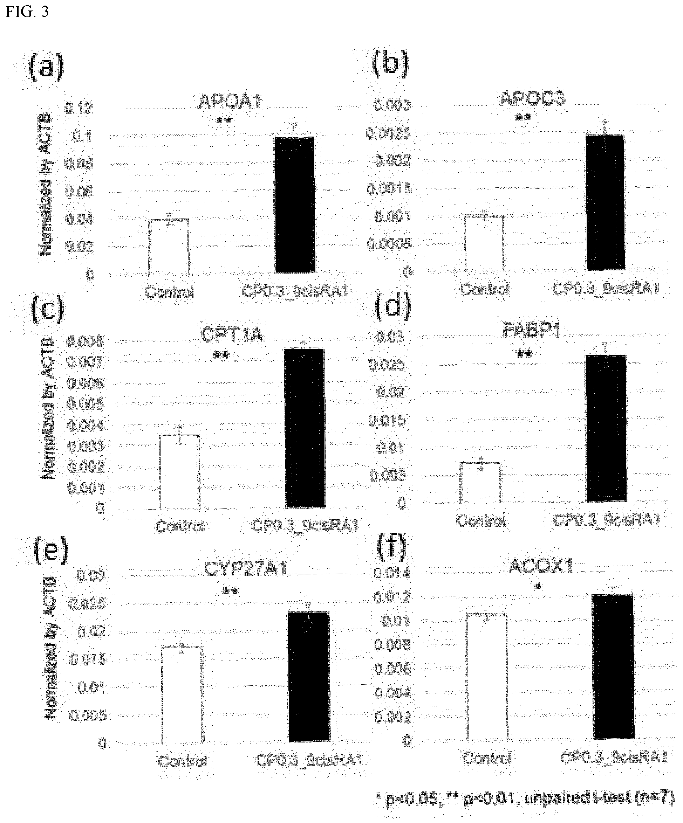

[FIG. 3] Figures 3a to 3f are a series of bar graphs showing expression levels of PPARA downstream gene markers (Figure 3a: APOA1, Figure 3b: APOC3, Figure 3c: CPT1A, Figure 3d: FABP1, Figure 3e: CYP27A1, and Figure 3f: ACOX1) in LTL-positive cells derived from kidney organoids cultured in the presence of a combination of CP775146 and 9-cis retinoic acid.

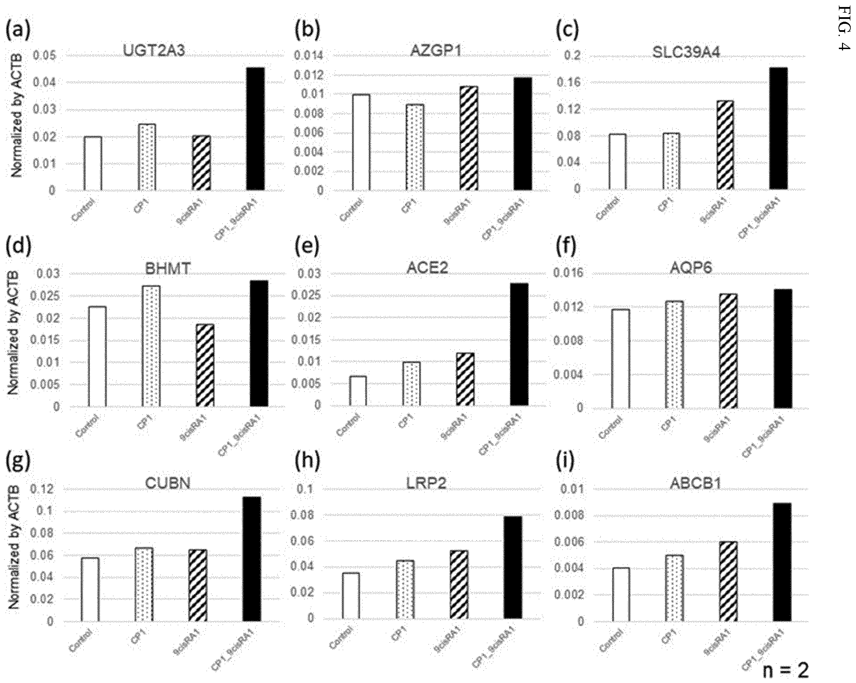

[FIG. 4] Figures 4a to 4i are a series of bar graphs showing expression levels of proximal tubule markers (Figure 4a: UGT2A3, Figure 4b: AZGP1, Figure 4c: SLC39A4, Figure 4d: BHMT, Figure 4e: ACE2, Figure 4f: AQP6, Figure 4g: CUBN, Figure 4h: LRP2, and Figure 4i: ABCB1) in LTL-positive cells derived from kidney organoids cultured in the presence of CP775146 singly, in the presence of 9-cis retinoic acid singly, or in the presence of a combination of CP775146 and 9-cis retinoic acid.

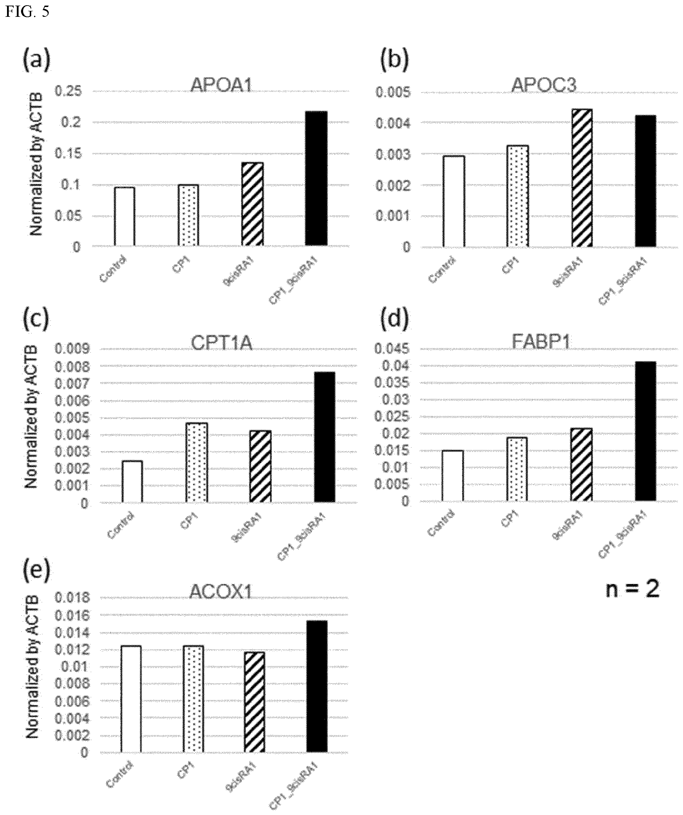

[FIG. 5] Figures 5a to 5e are a series of bar graphs showing expression levels of PPARA downstream gene groups (Figure 5a: APOA1, Figure 5b: APOC3, Figure 5c: CPT1A, Figure 5d: FABP1, and Figure 5e: ACOX1) in LTL-positive cells derived from kidney organoids cultured in the presence of CP775146 singly, in the presence of 9-cis retinoic acid singly, or in the presence of a combination of CP775146 and 9-cis retinoic acid.

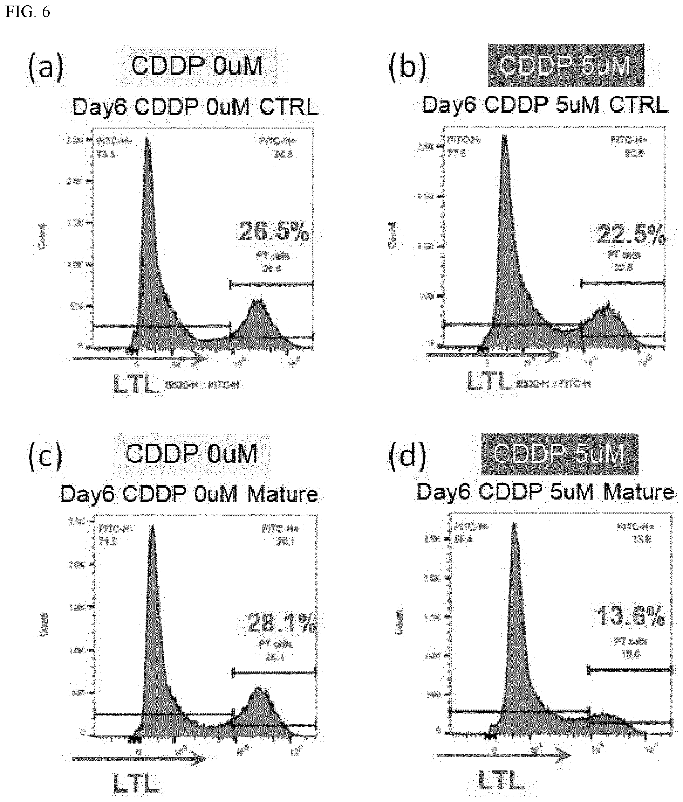

[FIG. 6] Figures 6a and 6b are 2D scatter plots of LTL-expressing cells derived from kidney organoids of the Control group cultured in the absence of cisplatin (CDDP) (Figure 6a) or in the presence of 5µ cisplatin (Figure 6b). Figures 6c and 6d are 2D scatter plots of LTL-positive cells derived from kidney organoids of the Mature group cultured in the absence of cisplatin (CDDP) (Figure 6a) or in the presence of 5µ cisplatin (Figure 6b).

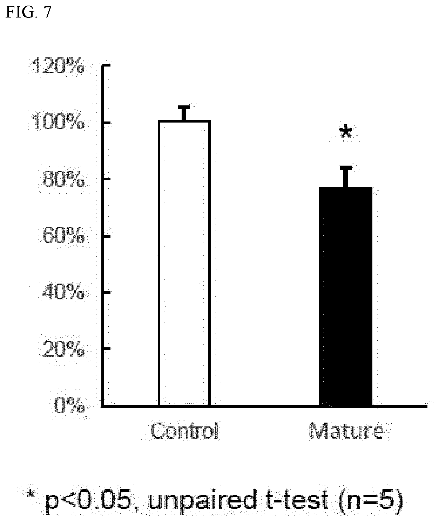

[FIG. 7] Figure 7 is a bar graph showing the results of a cisplatin nephrotoxicity test.

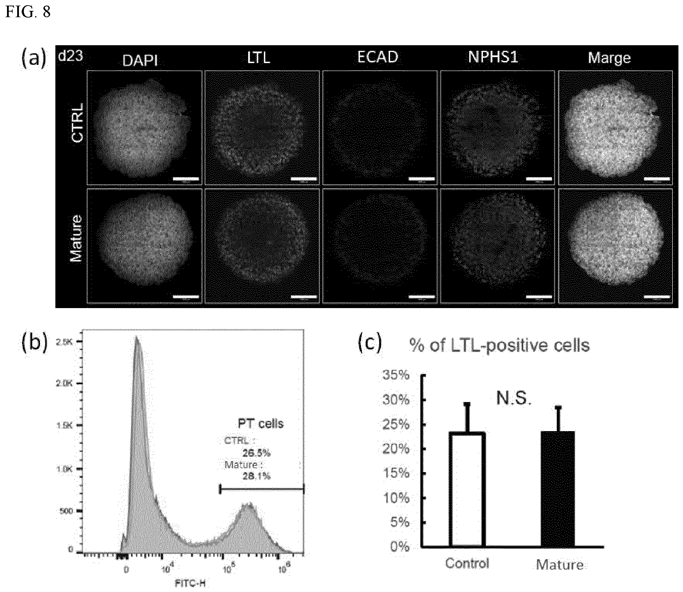

[FIG. 8] Figure 8a shows an immunostaining image of the kidney organoid. Figure 8b is a histogram of cells that constitutes the kidney organoid. Figure 8c is a bar graph showing the percentage of LTL-positive cells in the kidney organoid of the Control group and the percentage of LTL-positive cells in the kidney organoid of the Mature group.

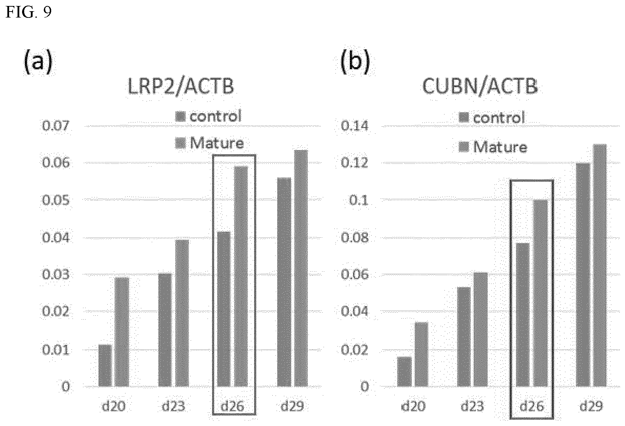

[FIG. 9] Figure 9a is a bar graph showing an expression level of LRP2. Figure 9b is a bar graph showing an expression level of CUBN.

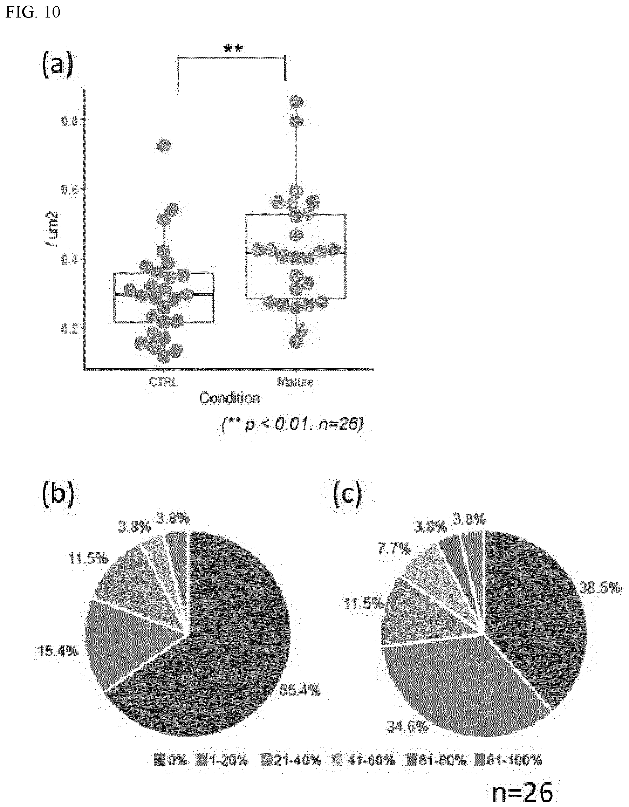

[FIG. 10] Figure 10a is a boxplot showing the size distribution of endosomes in the kidney organoid. Figure 10b is a pie chart showing the percentage of Large endosomes (≥0.5 µm²) to the total number of endosomes per cell in the kidney organoid of the Control group. Figure 10c is a pie chart showing the percentage of Large endosomes (≥0.5 µm²) to the total number of endosomes per cell in the kidney organoid of the Mature group.

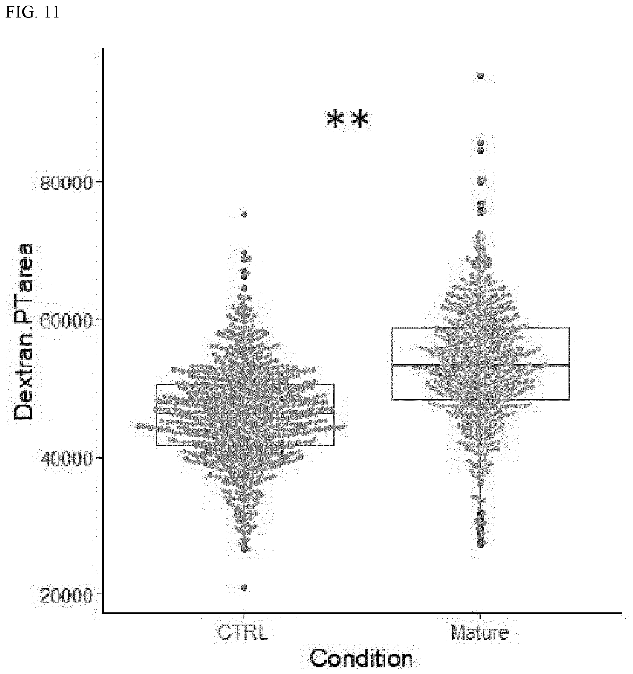

[FIG. 11] Figure 11 is a boxplot showing dextran uptake in the kidney organoid.

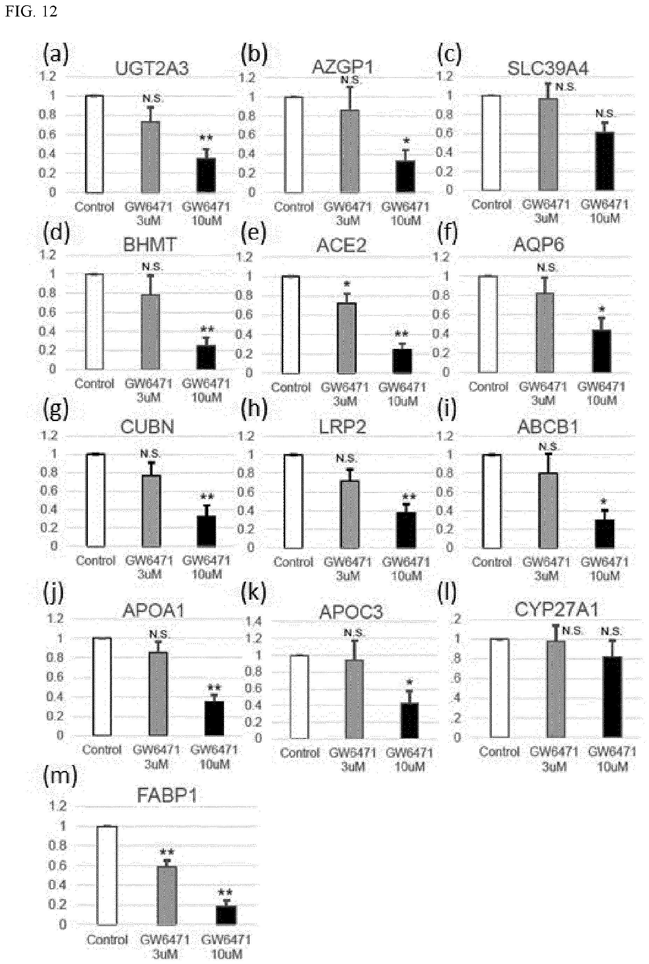

[FIG. 12] Figure 12 shows a series of bar graphs showing expression levels of maturation markers of proximal tubular cells in the kidney organoids created using PPAPA antagonists. Figure 12a is a bar graph showing an expression level of UGT2A3. Figure 12b is a bar graph showing an expression level of AZGP1. Figure 12c is a bar graph showing an expression level of SLC39A4. Figure 12d is a bar graph showing an expression level of BHMT. Figure 12e is a bar graph showing an expression level of ACE2. Figure 12f is a bar graph showing an expression level of AQP6. Figure 12g is a bar graph showing an expression level of CUBN. Figure 12h is a bar graph showing an expression level of LRP2. Figure 12i is a bar graph showing an expression level of ABCB1. Figure 12j is a bar graph showing an expression level of APOA1. Figure 12k is a bar graph showing an expression level of APOC3. Figure 121 is a bar graph showing an expression level of CYP27A1. Figure 12m is a bar graph showing an expression level of FABP1.

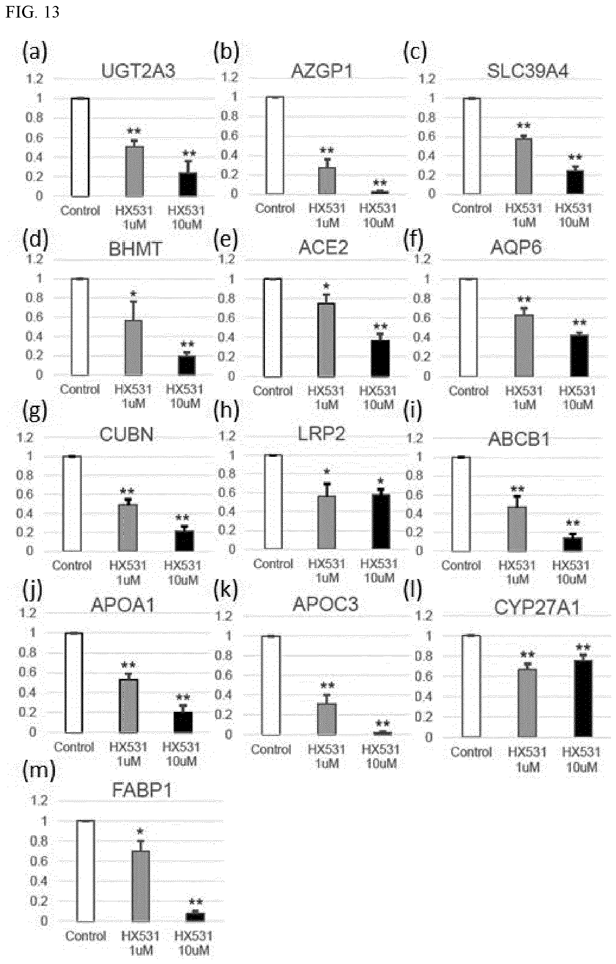

[FIG. 13] Figure 13 shows a series of bar graphs showing expression levels of maturation markers of proximal tubular cells in the kidney organoids prepared using RXR antagonists. Figure 13a is a bar graph showing an expression level of UGT2A3. Figure 13b is a bar graph showing an expression level of AZGP1. Figure 13c is a bar graph showing an expression level of SLC39A4. Figure 13d is a bar graph showing an expression level of BHMT. Figure 13e is a bar graph showing an expression level of ACE2. Figure 13f is a bar graph showing an expression level of AQP6. Figure 13g is a bar graph showing an expression level of CUBN. Figure 13h is a bar graph showing an expression level of LRP2. Figure 13i is a bar graph showing an expression level of ABCB1. Figure 13j is a bar graph showing an expression level of APOA1. Figure 13k is a bar graph showing an expression level of APOC3. Figure 131 is a bar graph showing an expression level of CYP27A1. Figure 13m is a bar graph showing an expression level of FABP1.

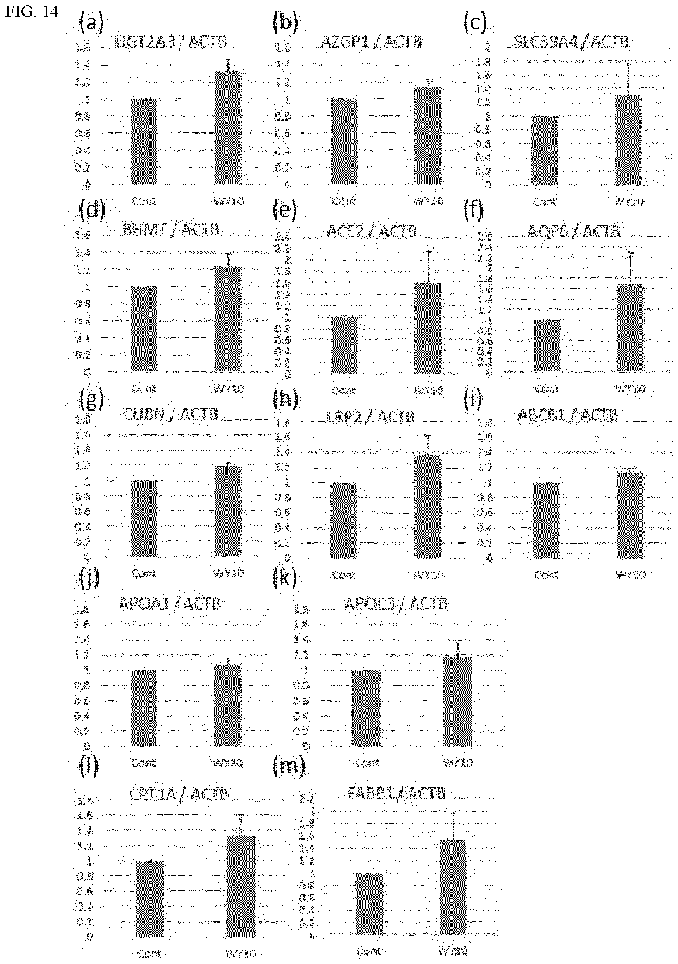

[FIG. 14] Figure 14 shows a series of bar graphs showing expression levels of maturation markers of proximal tubular cells in the kidney organoids prepared using pirinic acid. Figure 14a is a bar graph showing an expression level of UGT2A3. Figure 14b is a bar graph showing an expression level of AZGP1. Figure 14c is a bar graph showing an expression level of SLC39A4. Figure 14d is a bar graph showing an expression level of BHMT. Figure 14e is a bar graph showing an expression level of ACE2. Figure 14f is a bar graph showing an expression level of AQP6. Figure 14g is a bar graph showing an expression level of CUBN. Figure 14h is a bar graph showing an expression level of LRP2. Figure 14i is a bar graph showing an expression level of ABCB1. Figure 14j is a bar graph showing an expression level of APOA1. Figure 14k is a bar graph showing an expression level of APOC3. Figure 141 is a bar graph showing an expression level of CPT1A. Figure 14m is a bar graph showing an expression level of FABP1.

### DESCRIPTION OF EMBODIMENTS

### [Kidney organoid including matured proximal tubule cells]

One aspect of the present disclosure provides a kidney organoid including matured proximal tubule cells. One aspect of the present disclosure provides a kidney organoid including matured proximal tubule cells, wherein the kidney organoid expresses a proximal tubule marker and the proximal tubule marker is at least one selected from the group consisting of UGT2A3, AZGP1, SLC39A4, BHMT, ACE2, AQP6, CUBN, LRP2, ABCB1, SERPINA1, APOA1, and SPARCL1. In addition, one aspect of the present disclosure provides a kidney organoid including matured proximal tubule cells, wherein the kidney organoid may further express a PPARA downstream gene marker, and the PPARA downstream gene marker is at least one selected from the group consisting of APOA1, APOC3, CPT1A, FABP1, CYP27A1, and ACOX1.

The term "organoid" herein refers to a three-dimensional cell aggregate formed in vitro and resembling a biological tissue. The cell aggregate similar to a biological tissue is, for example, a cell aggregate with structures observed in the corresponding biological tissue. For example, the structure observed in a biological tissue may be a microscopically observable structure (for example, in the case of the kidney, glomerulus, or kidney tubules). Most of the cells constituting an organoid, for example, have the ability to differentiate and proliferate. The organoid is characterized, for example, by the expression of a marker gene (a transcription or translation product thereof). For example, a glomerulus can be identified by the expression of a glomerulus marker, such as Podocalyxin or Nephrin, or a combination thereof. For example, the proximal tubule can be identified, by the expression of a proximal tubule marker described below.

The term "early kidney organoid" herein refers to an organoid comprising one or more renal vesicles that are LHX1 positive. The early kidney organoid preferably includs cells that express the epithelial cell marker EpCAM. The renal vesicle is preferably LHX1 positive and JAG1 positive or CDH6 positive. The renal vesicle is preferably LHX1 positive, JAG1 positive, and CDH6 positive. The early kidney organoid can be produced from a pluripotent stem cell according to a known method, for example, the methods described in Takasato M., et al., Nat Cell Biol. 2014;16:118-26 (incorporated herein by reference) or Takasato M., et al., Nature. 2015;526:564-568 (incorporated herein by reference). The early kidney organoid can be produced from intermediate mesoderm cells, for example, according to a step B of the present disclosure, preferably according to the step B as described in Examples. The intermediate mesoderm cells can be produced from a pluripotent stem cell according to a step A of the present disclosure, preferably according to the step A described in Examples. The early kidney organoid can be produced from a pluripotent stem cell according to steps A and B of the present disclosure, preferably according to the steps A and B described in Examples.

The term "kidney organoid including matured proximal tubule cells" herein refers to a kidney organoid including cells expressing a proximal tubule marker described below. The kidney organoid including matured proximal tubule cells may have one or more glomeruli and/or tubules. In one example, the kidney organoid including matured proximal tubule cells further includes cells expressing a PPARA downstream genetic marker (preferably PPARA). The kidney organoid including matured proximal tubule cells include, for example, proximal tubules and glomeruli. The term "kidney organoid" herein refers to an organoid at any stage of maturation, from an early kidney organoid to a differentiated kidney organoid including matured proximal tubule cells. The kidney organoid also includes, for example, a known kidney organoid or a kidney organoid produced by a known method.

The term "matured proximal tubule cells" herein express a proximal tubule marker described below. In one example, matured proximal tubule cells express a PPARA downstream genetic marker described further below. Proximal tubular cells, for example, are present in the vicinity of the glomerulus in the kidney organoid. In one example, matured proximal tubule cells express the proximal tubule marker and the PPARA downstream gene marker.

An organoid expressing a particular marker (for example, a marker gene or marker protein) may be an organoid in which for example, the average expression level of a specific marker per cell in a specific cell population derived from the organoid is significantly greater than the average expression level of the specific marker per cell in the specific cell population derived from the control organoid. The term "significantly" in this context means, for example, that the p-value in a t-test is less than 0.05, preferably less than 0.01. Particular cell populations are, for example, proximal tubular cells. Proximal tubular cells that are a specific cell population derived from the organoid can be produced using, for example, MACS using the antibody against a specific marker (for example, LTL). Comparison of expression levels can be performed, for example, using at least three (for example, three, four, five, six, seven, or eight or more) organoids. In one example, for the organoid expressing a specific marker, the average expression level of a specific marker per cell in the specific cell population derived from the organoid is 1.2 times (for example, 1.2 times or more, 1.5 times or more, 1.8 times or more, 2.0 times or more, 2.3 times or more, or 2.5 times or more, or 1.2 times to 2.5 times, or 1.5 times to 2.5 times) higher than the average expression level of the specific marker per cell in the specific cell population derived from the control organoid. In one example, the organoid of interest is a kidney organoid including matured proximal tubule cells according to the present disclosure, and a control organoid is an early kidney organoid.

In one embodiment, for the kidney organoid including matured proximal tubule cells, an expression level of at least one proximal tubule marker selected from the group of proximal tubule markers is significantly higher than the expression level of the at least one proximal tubule marker in the early kidney organoid. In one embodiment, for the kidney organoid including matured proximal tubule cells, the expression level of at least one PPARA downstream gene marker selected from the group of PPARA downstream gene markers is significantly higher than the expression level of the at least one PPARA downstream gene marker in the early kidney organoid. In one embodiment, for the kidney organoid including matured proximal tubule cells, the expression level of at least one proximal tubule marker selected from the group of proximal tubule markers is significantly higher than the expression level of the at least one proximal tubule marker in the early kidney organoid, and/or the expression level of at least one PPARA downstream gene marker selected from the group of PPARA downstream gene markers is significantly higher than the expression level of the at least one PPARA downstream gene marker in the early kidney organoid.

For the kidney organoid according to the above embodiment, the expression level of the at least one proximal tubule marker in the kidney organoid is higher (for example, 1.2 times or more, 1.5 times or more, 1.8 times or more, 2.0 times or more, 2.3 times or more, or 2.5 times or more, or 1.2 to 2.5 times, or 1.5 to 2.5 times) than the expression level of the at least one proximal tubule marker in the early kidney organoid, and/or the expression level of the at least one PPARA downstream gene marker in the kidney organoid is higher (for example, 1.2 times or more, 1.5 times or more, 1.8 times or more, 2.0 times or more, 2.3 times or more, or 2.5 times or more, or 1.2 to 2.5 times, or 1.5 to 2.5 times) than the expression level of the at least one PPARA downstream gene marker in the early kidney organoid. In the above example, the "expression level is higher" may be "expression level is significantly higher."

The level of expression of the marker in an organoid of interest (for example, a kidney organoid including matured proximal tubule cells) and the level of expression of the marker in a control organoid (for example, an early kidney organoid) can be compared by using, for example, a plurality of (for example, at least two) markers. In this example, the organoid that expresses specific markers is an organoid in which the average expression level of a predetermined number (for example, six) or more of the plurality of (for example, 10) markers in the organoid of interest is higher than the average expression level in the control organoid. For example, the predetermined number may be the same (eight) as the number of markers to be measured (for example, eight), or may be no less than one fewer (for example, seven, six, five, four, three, two, or one). For example, when the number of markers to be measured is nine, the predetermined number is three or more, four or more, preferably five or more, and more preferably six or more.

For the kidney organoid according to the above embodiment, the number of proximal tubule markers having the expression level being higher than or equal to the expression level in the early kidney organoid is a predetermined number (for example, one, two, three, or four) or more, and/or the number of PPARA downstream gene markers having the expression level being higher than the expression level in the early kidney organoid is higher than or equal to a predetermined number (for example, one, two, three, or four). The predetermined number is appropriately set depending on the number of markers included in the group of proximal kidney tubule markers and the number of markers included in the group of PPARA downstream gene markers. The predetermined number is set in consideration of, for example, the types of markers included in the group of the above markers and the types of markers for which a significant difference or a tendency for the expression level to increase was observed in the examples of the present description. In one example, when a marker A, marker B, marker C, and marker D are included in the group of the above markers, and in the example, a significant difference or a tendency for the expression level to increase is observed for the marker A, marker B, and marker C, the predetermined number may be set to at least one (for example, one, two, or three), at least two (for example, two or three), or three. In the above example, the "expression level is higher" may be "expression level is significantly higher".

An organoid expressing a specific marker is, for example, an organoid including a predetermined proportion or more of cells expressing the specific marker. The "cells expressing a specific marker" in this context may be matured proximal tubule cell. The predetermined proportion may be, for example, 5% or more, 10% or more, 15% or more, 20% or more, or 25% or more of the cell population constituting the organoid. In one example, an organoid expressing a specific marker is an organoid including 5% or more (for example, 10% or more, 15% or more, 20% or more, or 25% or more) of cells expressing the specific marker at a predetermined level or more. The predetermined level is, for example, the third quartile of the expression level of a negative control cell population that does not express the specific marker.

In the above example, the predetermined marker is a proximal tubule marker described below and/or a PPARA downstream gene marker described below. The expression level of a marker may be the amount of a transcription product thereof, the amount of a translation product thereof, or the relative levels thereof. When the marker is a gene, the expression level of the marker may be the amount of a transcription product thereof, the amount of a translation product thereof, or the relative levels thereof. When the marker is a protein, the expression level of the marker may be the amount of a translation product thereof or the relative level thereof. The relative level means the amount of the marker relative to an endogenous control. As the endogenous control, for example, β-actin or glyceraldehyde-3-phosphate dehydrogenase (GAPDH) can be used. The relative level is, for example, the amount of marker relative to β-actin. The amount of a transcription product can be measured, for example, by quantitative PCR. The amount of a translation product can be measured, for example, by FACS or MACS. Such measurement can be performed by a fluorescent immunostaining method, fluorescence-activated cell sorting (FACS), or magnetic affinity cell sorting (MACS). FACS or MACS can be performed using commercially available FACS or MACS apparatus.

The term "LHX1" herein is an abbreviation for LIM homeobox 1, and means a protein encoded by the LHX1 gene. Cells expressing LHX1 can be detected, for example, by the immunostaining method with an anti-LHX1 antibody. LHX1-positive renal vesicles can be detected for the kidney organoid by the immunostaining method with an anti-LHX1 antibody.

The term "JAG1" herein is an abbreviation for Jagged1 protein encoded by the JAG1 gene, and means one of five cell surface proteins that interact with four receptors in the Notch signaling pathway. Cells expressing JAG1 can be detected, for example, by the immunostaining method with an anti-JAG1 antibody. JAG1-positive renal vesicles can be detected for the kidney organoid by the immunostaining method with an anti-JAG1 antibody.

The term "CDH6" herein means the cadherin 6 protein encoded by the CDH6 gene. Cells expressing CDH6 can be detected, for example, by the immunostaining method with an anti-CDH6 antibody. CDH6-positive renal vesicles can be detected for the kidney organoid by the immunostaining method with an anti-CDH6 antibody.

The term "EpCAM" as used in the present description is an abbreviation for epithelial cell adhesion molecule and refers to a transmembrane glycoprotein. EpCAM can be used as an epithelial cell marker. Cells expressing EpCAM can be detected, for example, by an immunostaining method with an anti-EpCAM antibody.

The "proximal tubule marker" herein is at least one selected from the group consisting of UGT2A3, AZGP1, SLC39A4, BHMT, ACE2, AQP6, CUBN, LRP2, ABCB1, SERPINA1, APOA1, and SPARCL1. The proximal tubule marker herein is also referred to as a proximal tubule maturation marker. The proximal tubule marker is, for example, at least one selected from the group consisting of UGT2A3, AZGP1, SLC39A4, BHMT, ACE2, AQP6, CUBN, LRP2, and ABCB1. The proximal tubule marker is, for example, at least one, two, three, four, five, six, seven, eight, nine, 10, 11, or 12 types selected from the group of proximal tubule markers. The proximal tubule marker is, for example, at least two selected from the group of proximal tubule markers (for example, two, three, four, five, six, seven, preferably eight, preferably nine, preferably 10, preferably 11, or more preferably 12 types). The proximal tubule marker is, for example, at least four types selected from the group of proximal tubule markers (for example, four, five, six, seven, preferably eight, preferably nine, preferably 10, preferably 11, or more preferably 12 types). The proximal tubule marker is, for example, at least six types selected from the group of proximal tubule markers (for example, six, seven, preferably eight, preferably nine, preferably 10, preferably 11, or more preferably 12 types). The proximal tubule marker, for example, at least seven types selected from the group of proximal tubule markers (for example, seven, preferably eight, preferably nine, preferably 10, preferably 11, or more preferably 12 types). The proximal tubule marker is, for example, eight, nine, 10, 11, or 12 types selected from the group of proximal tubule markers. The proximal tubule marker, for example, 10, 11, or 12 types selected from the group of proximal tubule markers. The proximal tubule marker is, for example, 11 or 12 types selected from the group of proximal tubule markers.

The proximal tubule marker is, for example, at least one type selected from the group consisting of UGT2A3, SLC39A4, ACE2, AQP6, CUBN, LRP2, ABCB1, SERPINA1, APOA1, and SPARCL1 (for example, one, two, three, four, five, six, seven, eight, nine, or 10 types). The proximal tubule marker is, for example, at least two types selected from the group of the proximal tubule markers (for example, two, three, four, five, preferably six, preferably seven, preferably eight, preferably nine, or more preferably 10 types). The proximal tubule marker is, for example, at least four types selected from the group of the proximal tubule markers (for example, four, five, preferably six, preferably seven, preferably eight, preferably nine, or more preferably 10 types). The proximal tubule marker is, for example, at least six, seven, eight, nine, or 10 types selected from the group of the proximal tubule markers. The proximal tubule marker is, for example, at least eight, nine, or 10 types selected from the group of the proximal tubule markers. The proximal tubule marker is, for example, at least nine or 10 types selected from the group of the proximal tubule markers.

The proximal tubule marker is, for example, at least one type selected from the group consisting of UGT2A3, SLC39A4, ACE2, AQP6, CUBN, LRP2, and ABCB1 (for example, one, two, three, four, five, six, or seven types). The proximal tubule marker is, for example, at least two types selected from the group of the proximal tubule markers (for example, two, three, four, five, preferably six, or more preferably seven types). The proximal tubule marker is, for example, at least four types selected from the group of the proximal tubule markers (for example, four, five, preferably six, or more preferably seven types). The proximal tubule marker is, for example, at least six or seven types selected from the group of the proximal tubule markers.

The proximal tubule marker is, for example, at least one type selected from the group consisting of SLC39A4, ACE2, AQP6, CUBN, LRP2, ABCB1, and APOA1 (for example, one, two, three, four, five, six, or seven types). The proximal tubule marker is, for example, at least two types selected from the group of the proximal tubule markers (for example, two, three, four, preferably five, preferably six, or more preferably seven types). The proximal tubule marker is, for example, at least four types selected from the group of the proximal tubule markers (for example, four, preferably five, preferably six, or more preferably seven types). The proximal tubule marker is, for example, at least five, six, or seven types selected from the group of the proximal tubule markers. The proximal tubule marker is, for example, at least six or seven types selected from the group of the proximal tubule markers.

The proximal tubule marker is, for example, at least one type selected from the group consisting of SLC39A4, ACE2, AQP6, CUBN, LRP2, and ABCB1 (for example, one, two, three, four, five, or six types). The proximal tubule marker is, for example, at least two types selected from the group of the proximal tubule markers (for example, two, three, four, preferably five, or more preferably six types). The proximal tubule marker is, for example, at least four types selected from the group of the proximal tubule markers (for example, four, preferably five, or more preferably six types). The proximal tubule marker is, for example, at least five or six types selected from the group of the proximal tubule markers.

The proximal tubule marker is, for example, at least one type selected from the group consisting of UGT2A3, SLC39A4, ACE2, CUBN, LRP2, ABCB1, and APOA1 (for example, one, two, three, four, five, six, or seven types). The proximal tubule marker is, for example, at least two types selected from the group of the proximal tubule markers (for example, two, three, four, preferably five, preferably six, or more preferably seven types). The proximal tubule marker is, for example, at least four types selected from the group of the proximal tubule markers (for example, four, preferably five, preferably six, or more preferably seven types). The proximal tubule marker is, for example, at least five, six, or seven types selected from the group of the proximal tubule markers. The proximal tubule marker is, for example, at least six or seven types selected from the group of the proximal tubule markers.

The proximal tubule marker is, for example, at least one type selected from the group consisting of UGT2A3, SLC39A4, ACE2, CUBN, LRP2, and ABCB1 (for example, one, two, three, four, five, or six types). The proximal tubule marker is, for example, at least two types selected from the group of the proximal tubule markers (for example, two, three, four, preferably five, or more preferably six types). The proximal tubule marker is, for example, at least four types selected from the group of the proximal tubule markers (for example, four, preferably five, or more preferably six types). The proximal tubule marker is, for example, at least five or six types selected from the group of the proximal tubule markers.

The term "LTL" is an abbreviation for Lotus Tetragonolobus Lectin, and refers to a glycoprotein that can exhibit specificity for α-linked L-fucose containing oligosaccharides. The term "UGT2A3" refers to UDP-Glucuronosyltransferase 2A3. The term "AZGP1" is an abbreviation for Zinc-alpha2-glycoprotein. The AZGP1 gene encodes zinc-α2-glycoprotein (ZAG). The term "SLC39A4" refers to Solute Carrier Family 39 Member 4, and the SLC39A4 gene encodes Zinc/Iron-regulated transporter-like Protein 4 (ZIP4). The term "BHMT" is an abbreviation for Betaine-Homocysteine S-Methyltransferase. The BHMT gene encodes an enzyme that uses betaine as a methyl donor and catalyzes the remethylation of homocysteine (Hcy).

The term "ACE2" is an abbreviation for Angiotensin-Converting Enzyme 2. The ACE2 gene encodes an isoform of angiotensin-converting enzyme (ACE). The term "AQP6" is an abbreviation for Aquaporin6. The AQP6 gene encodes an aquaporin protein that functions as a water channel in cells. The term "CUBN" is an abbreviation for Cubilin. The CUBN gene encodes Cubilin, which functions as an intrinsic factor-vitamin B12 complex. The term "LRP2" is an abbreviation for LDL Receptor Related Protein 2, which is a protein belonging to a receptor family that has structural similarity to the low-density lipoprotein receptor (LDLR). The LRP2 gene encodes LDL Receptor Related Protein 2. The term "ABCB1" is an abbreviation for ABC transporter subfamily B member 1. The ABCB1 gene encodes P-glycoprotein 1 (also referred to as ABCB1 or MDR1), a member of the superfamily of ATP-binding cassette (ABC) transporters.

The term "SERPINA1" is an abbreviation for serpin family A member 1. The SERPINA1 gene encodes SERPINA1, a prototypical member of the Serpin superfamily of serine protease inhibitors. The term "APOA1" is an abbreviation for Apolipoprotein A-I and refers to the major protein component of HDL particles in plasma. The APOA1 gene encodes Apolipoprotein A-I. The term "SPARCL1" is an abbreviation for SPARC-like protein 1, and is a matrix protein also known as Hevin. The SPARCL1 gene encodes the matrix protein SPARCL1.

The "PPARA downstream gene marker" herein is at least one type selected from the group consisting of APOA1, APOC3, CPT1A, FABP1, CYP27A1, and ACOX1. The PPARA downstream gene marker is, for example, one, two, three, four, five, or six types selected from the group of the PPARA downstream gene markers. The PPARA downstream gene marker is at least two types selected from the group of the PPARA downstream gene markers (for example, two, three, four, preferably five, or more preferably six types). The PPARA downstream gene marker is at least three types selected from the group of the PPARA downstream gene markers (for example, three, four, preferably five, or more preferably six types). The PPARA downstream gene marker is at least four types, preferably five types, or more preferably six types selected from the group of the PPARA downstream gene markers.

The PPARA downstream gene marker is, for example, at least one type selected from the group consisting of APOA1, APOC3, CPT1A, FABP1, and CYP27A1 (for example, one, two, three, preferably four, or more preferably five types). The PPARA downstream gene marker is at least two types selected from the group of the PPARA downstream gene markers (for example, two, three, preferably four, or more preferably five types). The PPARA downstream gene marker is four or five types selected from the group of the PPARA downstream gene markers.

The PPARA downstream gene marker is, for example, at least one type selected from the group consisting of APOA1, CPT1A, FABP1, ACOX1, and CYP27A1 (for example, one, two, three, preferably four, or more preferably five types). The PPARA downstream gene marker is, for example, at least two types selected from the group of the PPARA downstream gene markers (for example, two, three, preferably four, or more preferably five types). The PPARA downstream gene marker is, for example, four or five types selected from the group of the PPARA downstream gene markers.

The PPARA downstream gene marker is, for example, at least one type selected from the group consisting of APOA1, CPT1A, FABP1, and ACOX1 (for example, one, two, preferably three, or more preferably four types). The PPARA downstream gene marker is, for example, at least two types selected from the group of the PPARA downstream gene markers (for example, two, preferably three, or more preferably four types). The PPARA downstream gene marker is, for example, three or four types selected from the group of the PPARA downstream gene markers.

The PPARA downstream gene marker is, for example, at least one type selected from the group consisting of APOA1, CPT1A, and FABP1 (for example, one, preferably two, or more preferably three types). The PPARA downstream gene marker is, for example, two or three types selected from the group of the PPARA downstream gene markers.

The term "APOA1" is an abbreviation for Apolipoprotein A-I and refers to the major protein component of HDL particles in plasma. The APOA1 gene encodes Apolipoprotein A-I. The term "APOC3" is an abbreviation for Apolipoprotein C-III and refers to a relatively small protein composed of 79 amino acids. The APOC3 gene encodes Apolipoprotein C-III. The term "CPT1A" is an abbreviation for Carnitine palmitoyltransferase IA, and is an enzyme involved in the outer mitochondrial membrane. The CPT1A gene encodes Carnitine palmitoyltransferase IA.

The term "FABP1" is an abbreviation for Fatty Acid Binding Protein 1. The FABP1 gene encodes Fatty Acid Binding Protein 1, also known as liver-type fatty acid binding protein. The term "CYP27A1" refers to the gene encoding cytochrome P450 oxidase, also known as sterol 27-hydroxylase. The term "ACOX1" is an abbreviation for Acyl-CoA Oxidase 1. The ACOX1 gene encodes peroxisomal acyl-coenzyme A oxidase 1.

Matured proximal tubule cells, for example, express at least one type selected from the group of the PPARA downstream gene markers according to the present disclosure. Matured proximal tubule cells, for example, express at least one type selected from the group of proximal tubule markers according to the present disclosure. Matured proximal tubule cells, for example, express at least one type selected from the group of the PPARA downstream gene markers according to the present disclosure, and expresses at least one type selected from the group of the proximal tubule markers according to the present disclosure. Matured proximal tubule cells, for example, express at least three markers selected from the group of the PPARA downstream gene markers according to the present disclosure (for example, three, four, preferably five, or more preferably six types), and expresses at least five markers selected from the group of the proximal tubule markers according to the present disclosure (for example, five, six, seven, preferably eight, or more preferably nine types).

The kidney organoid including matured proximal tubule cells expresses at least one type selected from the group of the proximal tubule markers according to the present disclosure. In one example, the kidney organoid including matured proximal tubule cells further expresses at least one type selected from the group of the PPARA downstream gene markers according to the present disclosure. The kidney organoid including matured proximal tubule cells expresses, for example, at least one type selected from the group of the proximal tubule markers according to the present disclosure, and expresses at least one type selected from the group of the proximal tubule markers according to the present disclosure. Expression of the PPARA downstream gene markers in the kidney organoid including proximal tubular cells can be detected based on the transcription product of the marker gene or the translation product of the marker gene. In addition, expression of the proximal tubule markers in the kidney organoid including proximal tubular cells can be detected based on the transcription product of the marker gene or the translation product of the marker gene. The transcription product of the marker gene can be measured, for example, by quantitative PCR. The translation product of the marker gene can be measured, for example, by a fluorescent immunostaining method or by FACS or MACS.

The PPARA downstream gene marker in the kidney organoid including matured proximal tubule cells may be evaluated as being expressed if the expression level (for example, the amount of transcription product of the marker gene) is significantly greater than the expression level of the corresponding PPARA downstream gene marker in the kidney organoid (control organoid) cultured using a medium substantially free of either or both of an RXR agonist and a PPAR agonist. Preferably, the expression level of the PPARA downstream gene marker is evaluated as being expressed if significantly greater than the expression level of the corresponding PPARA downstream gene marker in the control organoid cultured using a medium substantially free of an RXR agonist or a PPAR agonist. In addition, the proximal tubule marker in the kidney organoid including matured proximal tubule cells may be evaluated as being expressed, if the expression level (for example, the amount of transcription product of the marker gene) is significantly greater than the expression level of the corresponding proximal tubule marker in the kidney organoid (control organoid) cultured using a medium substantially free of either or both of an RXR agonist and a PPAR agonist. Preferably, the expression level of the proximal tubule marker is evaluated to be expressed if significantly greater than the expression level of the corresponding proximal tubule marker in the control organoid cultured using medium substantially free of an RXR agonist or a PPAR agonist.

The medium substantially free of either or both an RXR agonist and a PPAR agonist may be, for example, a medium containing an RXR agonist and a PPAR agonist (for example, induction medium C described below) used to induce the kidney organoid including matured proximal tubule cells of interest, and a medium with the same composition and/or dose except that the medium is free of substantially either or both of an RXR agonist and a PPAR agonist. "Substantially free" in this context does not exclude complete absence of either an RXR agonist or a PPAR agonist. In one example, the medium substantially free of an RXR agonist or a PPAR agonist may include, for example, less than 10 nM, less than 5 nM, or less than 1 nM of the PPAR agonist and less than 10 nM, less than 5 nM, or less than 1 nM of the RXR agonist. In one example, the medium that is substantially free of an RXR agonist and a PPAR agonist is completely free of the RXR agonist and the PPAR agonist.

In other embodiments, the PPARA downstream gene marker in the kidney organoid including matured proximal tubule cells may be evaluated as being expressed if the expression level (for example, the amount of the transcription product of the marker gene) is significantly greater than the expression level of the corresponding PPARA downstream gene marker in the early kidney organoid (control organoid). In addition, the proximal tubule marker in the kidney organoid including matured proximal tubule cells may be evaluated as being expressed if the expression level (for example, the amount of the transcription product of the marker gene) is significantly greater than the expression level of the corresponding proximal tubule marker in the early kidney organoid (control organoid).

### [Method for producing kidney organoid including matured proximal tubule cells]

One aspect of the present disclosure provides a method for producing a kidney organoid including matured proximal tubule cells, the method including culturing an early kidney organoid with a culture medium C containing an RXR agonist and a PPAR agonist.

One embodiment of the present disclosure provides a method for producing a kidney organoid including matured proximal tubule cells, the method including: culturing intermediate mesoderm cells with an induction medium B (induction medium b2) containing a fibroblast growth factor to form an intermediate mesoderm spheroid, and culturing the intermediate mesoderm spheroid with an induction medium A (induction medium a2) containing a GSK3β inhibitor, then with an induction medium B (induction medium b3) containing a fibroblast growth factor to induce an early kidney organoid (step B); and culturing the early kidney organoid with an induction medium C containing an RXR agonist and a PPAR agonist (Step C).

One embodiment of the present disclosure provides a method for producing a kidney organoid including matured proximal tubule cells, the method including: culturing a pluripotent stem cell to induce intermediate mesoderm cells (step A); culturing the intermediate mesoderm cells using the induction medium B (induction medium b2) containing a fibroblast growth factor to form intermediate mesoderm spheroids, and culturing the intermediate mesoderm spheroid with the induction medium A (induction medium a2) containing a GSK3β inhibitor, then with an induction medium B (induction medium b3) containing a fibroblast growth factor to induce an early kidney organoid (step B); and culturing the early kidney organoid with the induction medium C containing an RXR agonist and a PPAR agonist (step C).

### Step A: Induction of differentiation of intermediate mesoderm cells

Step A includes culturing a pluripotent stem cell to induce intermediate mesoderm cells. More specifically, the step A includes culturing a pluripotent stem cell with an induction medium A (induction medium a1) containing a GSK3β inhibitor, then with an induction medium B (induction medium b1) containing a fibroblast growth factor to induce intermediate mesoderm cells.

Culturing a pluripotent stem cell with the induction medium a1 can be performed under a known cell culture condition. Known cell culture conditions may include maintaining cells at 37°C and 5% CO₂. The culture temperature is not limited to 37°C, and any temperature known in the field of cell culture can be used as appropriate. The CO₂ concentration is not limited to 5%, and any CO₂ concentration known in the cell culture field can be used as appropriate. Culturing a pluripotent stem cell with the induction medium A can be performed for two to eight days, three to seven days, four to six days, or five days.

The step A includes culturing a pluripotent stem cell with the induction medium a1, then with the induction medium b1 containing a fibroblast growth factor. Culturing with the induction medium b1 can be performed, for example, after culturing with the induction medium a1 causes colonies composed of pluripotent stem cells to spread both horizontally and vertically to the culture surface and to form a rounded shape. Culturing rounded cell colonies with the induction medium B containing a fibroblast growth factor can induce intermediate mesoderm cells.

Culturing the round-shaped cell colony (culture of pluripotent stem cells with the induction medium a1) with the induction medium b1 can be performed under a known cell culture condition. Known cell culture conditions may include maintaining cells at 37°C and 5% CO₂. The culture temperature is not limited to 37°C, and any temperature known in the field of cell culture can be used as appropriate. The CO₂ concentration is not limited to 5%, and any CO₂ concentration known in the cell culture field can be used as appropriate. Culturing the round-shaped cell colony with the induction medium B can be performed for two to six days, two to five days, two to four days, or three days. Due to this culture, the cell colony can change from a rounded shape to a sheet-like shape.

The term "pluripotent stem cell" herein refers to a stem cell that can be cultured in vitro and has the ability to differentiate into tissues derived from the three germ layers (ectoderm, mesoderm, and endoderm), that is, pluripotency. Pluripotent stem cells can be established from, for example, fertilized eggs, cloned embryos, reproductive stem cells, or stem cells in tissues. Pluripotent stem cells include, for example, embryonic stem cells (ES cells), induced pluripotent stem cells (iPS cells) derived from somatic cells, and embryonic tumor cells (EC cells), or embryonic germ stem cells (EG cells). Pluripotent stem cells are preferably ES cells or iPS cells.

The term "ES cell" herein is a stem cell that has self-renewal ability and pluripotency, and refers to a pluripotent stem cell derived from an early embryo. The ES cells are, for example, human ES cells.

The term "iPS cell" herein is a pluripotent stem cell derived from somatic cells, and refers to a cell that has been artificially given pluripotency similar to embryonic stem cells by reprogramming somatic cells. iPS cells can be established, for example, by reprogramming differentiated cells such as fibroblasts by expressing genes such as Oct3/4, Sox2, Klf4, and Myc. iPS cells are, for example, human iPS cells established by reprogramming differentiated cells such as human fibroblasts.

An "induction medium A" herein includes a basal medium and an additive containing a GSK3β inhibitor. The induction medium A used in the step A is also particularly referred to as the induction medium a1. The induction medium A used in the step B (particularly step b2) is also particularly referred to as the induction medium a2. In the present description, the explanation regarding the induction medium A also applies to the induction medium a1 and the induction medium a2, unless otherwise specified. The induction medium A can be prepared, for example, by adding the additive (solid or liquid) to a basic medium (liquid). The concentration of the additive added to the induction medium A is appropriately set by a person skilled in the art, taking into account the animal species that provided the cells used for culture. A "basic medium" may be a cell culture medium that can be prepared according to known protocols, or may be a commercially available cell culture medium. The basic medium may be, for example, Dulbecco's Modified Eagle Medium (DMEM), Minimum Essential Medium (MEM), Basic Medium Eagle (BME), a known stem cell medium, or a known medium for differentiating stem cells. The basic medium is preferably a medium for differentiating stem cells and may be, for example, STEMdiff APEL2 medium (STEMCELL Technologies). The medium for differentiating stem cells can be prepared, for example, in Nature Protocols, vol. 3, No. 5, pp. 768-776, 2008. The induction medium A may further contain protein-free medium (for example, PFHM-II), antibiotics (for example, penicillin-streptomycin, gentamicin), or antibiotic-antimycotic mixtures (for example, Antibiotic-Antimycotic), or a combination thereof.

The term "GSK3β inhibitor" herein refers to a compound that inhibits the action of serine-threonine protein kinase 3β, which is involved in various signal transduction pathways, including the wnt/p-catenin pathway. The GSK3β inhibitor may be, for example, CHIR-99021, SB216763, CHIR-98014, Staurosporine, K252A, WNT (preferably WNT3A) or TWS119, or a combination thereof. The GSK3β inhibitor is preferably CHIR-99021 or WNT (preferably WNT3A). The induction medium a1 may contain a GSK3β inhibitor other than CHIR-99021 at a concentration that exhibits an effect comparable to the GSK3β inhibitory effect exhibited by CHIR-99021 at 1 to 50 µM, 2 to 25 µM, 5 to 15 µM, 5 to 8 µM, or 8 µM. The induction medium a2 may contain a GSK3β inhibitor other than CHIR-99021 at a concentration that exhibits an effect comparable to the GSK3β inhibitory effect exhibited by CHIR-99021 at 1 to 50 µM, 2 to 25 µM, 5 to 15 µM, 5 to 10 µM, or 10 µM.

The "GSK3β inhibitory effect" can be measured, for example, based on transcriptional activity due to nuclear translocation of β-catenin in the presence of a predetermined amount of a GSK3β inhibitor. Transcriptional activity due to nuclear translocation of β-catenin can be measured according to known methods (for example, luciferase activity measurement). Transcriptional activity due to nuclear translocation of β-catenin can be measured, for example, using a commercially available kit (for example, LEADING LIGHT^{®} Wnt Reporter Assay Starter kit). The term "comparable effect" herein means an effect with an error of plus or minus 30%, 20%, or 10% compared to a control effect. In one embodiment, a comparable effect is an effect with an error plus or minus 30% compared to a control effect.

The induction medium A may contain 1 to 50 µM, 2 to 25 µM, or 5 to 15 µM of a GSK3β inhibitor (preferably CHIR-99021). The induction medium A may contain a GSK3β inhibitor at a concentration that exhibits an effect comparable to the GSK3β inhibitory effect exhibited by CHIR-99021 at 1 to 50 µM, 2 to 25 µM, or 5 to 15 µM. The induction medium A may contain 1 to 2000 ng/ml, 2 to 1000 ng/ml, or 5 to 400 ng/ml of WNT (preferably WNT3A) as a GSK3β inhibitor. When the induction medium A contains WNT (preferably WNT3A) as a GSK3β inhibitor, the induction medium a1 may contain WNT (preferably WNT3A) at a concentration that exhibits an effect comparable to the GSK3β inhibitory effect exhibited by CHIR-99021 at 1 to 50 µM, 2 to 25 µM, 5 to 15 µM, or 8 µM. The induction medium a2 may contain WNT (preferably WNT3A) at a concentration that exhibits an effect comparable to the GSK3β inhibitory effect exhibited by CHIR-99021 at 1 to 50 µM, 2 to 25 µM, 5 to 15 µM, or 10 µM.

An "induction medium B" herein includes a basic medium and an additive containing a fibroblast growth factor and substantially free of an RXR agonist. The induction medium B used in the step A is also particularly referred to as the induction medium b1. The induction medium B used in the step b1 is also particularly referred to as an induction medium b2. The induction medium B used in the step b3 is also particularly referred to as an induction medium b3. In the present description, the explanation regarding the induction medium B also applies to the induction medium b1, induction medium b2, and induction medium b3, unless otherwise specified. In one example, the additive for the induction medium B is also substantially free of a PPAR agonist. The additive for the induction medium B contains, for example, a fibroblast growth factor and is substantially free of an RXR agonist and a PPAR agonist. The induction medium B can be prepared, for example, by adding the additive (solid or liquid) to a basic medium (liquid). For the basic medium related to the induction medium B, the explanation regarding the basic medium related to the induction medium A applies as appropriate. The concentration of the additive added to the induction medium B is appropriately set by a person skilled in the art, taking into consideration the animal species that are provided with the cells used for culture. The induction medium B may further contain heparin, protein-free media (for example, PFHM-II), antibiotics (for example, penicillin-streptomycin, gentamicin), or antibiotic-antimycotic mixtures (for example, Antibiotic-Antimycotic), or a combination thereof. The induction medium b2 used to form spheroids from intermediate mesoderm cells may further contain an ROCK inhibitor (for example, Y-27632).

The term "substantially free of an RXR agonist" in the context of the induction medium B means, except for being completely free of an RXR agonist in the induction medium B, containing an RXR agonist in an amount such that there is differentiated and induced into an organoid possessing the same degree of maturity as the early kidney organoid formed in the step B with a medium with the same composition and/or dose. An "organoid possessing the same degree of maturity as the early kidney organoid" has one or more renal vesicles that are LHX1 positive, and contains less than 5% (preferably less than 4%, less than 3%, less than 2%, less than 1%, or less than 0.5%) of wolffian duct cells relative to the total cells derived from the organoid. The renal vesicle is preferably LHX1 positive and JAG1 positive or CDH6 positive. The renal vesicle is preferably LHX1 positive, JAG1 positive, and CDH6 positive. A "wolffian duct cell" is a CDH1-positive, PAX2-positive, and GATA3-positive cell. The proportion of wolffian duct cells to total cells derived from the organoid can be measured, for example, by single cell proteomic analysis (for example, single cell RNA sequencing). Being substantially free of an RXR agonist does not exclude being completely free of an RXR agonist. In one example, the induction medium B substantially free of an RXR agonist contains less than 10 pM, less than 5 pM, less than 1 pM, less than 0.5 pM, or less than 0.1 pM of an RXR agonist (for example, 9cis retinoic acid). The induction medium B substantially free of an RXR agonist is preferably completely free of an RXR agonist (for example, 9cis retinoic acid).

In another example, the induction medium B substantially free of an RXR agonist is substantially free of a PPAR agonist (including, for example, at least one selected from the group consisting of a PPARA agonist, PPARG agonist, PPARD agonist, PPARA/G agonist, PPARA/D agonist, PPARG/D agonist, and PPARA/G/D agonist). In the context of the induction medium B, the term "substantially free of an RXR agonist or PPAR agonist" means, except for being completely free of RXR and PPAR agonists in the induction medium B, containing an RXR agonist and/or PPAR agonist in an amount such that there is differentiated and induced into an organoid possessing the same degree of maturity as the early kidney organoid formed in the step B with a medium with the same composition and/or dose. In one example, the induction medium B substantially free of an RXR agonist and PPAR agonist contains less than 10 pM, preferably less than 5 pM, less than 1 pM, less than 0.5 pM, or less than 0.1 pM of an RXR agonist and/or PPAR agonist. The induction medium B substantially free of an RXR agonist and PPAR agonist is preferably completely free of an RXR agonist (for example, 9cis retinoic acid) and a PPAR agonist (for example, PPARA agonist).

The term "CDH1" herein refers to a gene encoding cadherin 1 protein. CDH1-positive cells are cells in which a CDH1 transcription product is produced. CDH1-positive cells can be detected by quantitative PCR with reverse transcriptase or single cell proteome analysis.

The term "PAX2" herein is an abbreviation for paired box gene 2, and refers to a gene encoding a homeobox transcription factor. PAX-positive cells are cells in which a PAX2 transcription product is produced. PAX2-positive cells can be detected by quantitative PCR with reverse transcriptase or single-cell proteome analysis.

The term "GATA3" herein refers to a gene encoding GATA3 transcription factor. GATA3-positive cells are cells in which a GATA3 transcription product is produced. GATA3-positive cells can be detected by quantitative PCR with reverse transcriptase or single cell proteome analysis.

CDH1-positive, PAX2-positive, and GATA3-positive cells are cells in which a CDH1 transcription product, a PAX2 transcription product, and a GATA3 transcription product are produced. The cells can be detected by single cell proteome analysis.

The term "fibroblast growth factor (FGF)" herein refers to a protein having 16,000 to 20,000 in molecular weight that promotes the proliferation of fibroblasts or endothelial cells. FGF include, for example, FGF1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, FGF22, or FGF23, or may be a combination thereof. FGF may be, for example, FGF9 singly or a combination of FGF9 and other FGFs (for example, FGF4). FGF is preferably FGF9 singly. The induction medium B may contain 20 to 1000 ng/mL, 60 to 500 ng/mL, or 100 to 300 ng/mL of FGF (preferably FGF9). The induction medium B may contain FGF other than FGF9 at a concentration that exhibits an effect comparable to the fibroblast proliferation effect exhibited by FGF9 at 20 to 1000 ng/mL, 60 to 500 ng/mL, or 100 to 300 ng/mL.

The term "intermediate mesoderm" herein means a sheet-like cell colony formed by culturing, using the induction medium B (induction medium b1), a round-shaped cell colony formed by culturing a pluripotent stem cell with the induction medium A (induction medium a1). The term "intermediate mesoderm cells" herein can be produced by culturing a pluripotent stem cell with the induction medium A (induction medium a1), then with the induction medium B (induction medium b1). The intermediate mesoderm cells can be produced, for example, by culturing a pluripotent stem cell with the induction medium A (induction medium a1) for two to eight days, three to seven days, four to six days, or five days, then with the induction medium B (induction medium b1) for two to six days, two to five days, two to four days, or three days.

The step A can be performed, for example, according to the following procedure. Human iPS or ES cells cultured on iMatrix-511 coating using StemFit AK02N medium (REPROCELL) are detached with TrypLE Select (Thermo Fisher Scientific Inc.). The detached cells are suspended in StemFit AK02N (10 µM Y-27632), and iMatrix-511 (Nippi Inc.) is added so as to provide a final concentration of 0.25 µg/cm². This cell suspension is seeded on a 12-well plate so as to provide 10,000 to 15,000 cells/cm², and cultured for one day at 37°C and 5% CO₂. Then the seeded cells are cultured for three to five days to induce a primitive streak by using STEMdiff APEL2medium (STEMCELL Tecnologies) (8 µM CHIR99021, 1% PFHM II, Antibiotic-Antimycotic, below the additives to the basic medium are written in parentheses), which is the induction medium A (induction medium a1). Medium exchange is performed every day from the fourth day onwards. Culturing for five days can increase the proportion of intermediate mesoderm cells, thereby allowing the formation of many proximal tubules. The cells cultured with the induction medium A are cultured for three to five days to induce intermediate mesoderm cells by using STEMdiff APEL2 medium (200 ng/mL FGF9, 1 µg/mL Heparin, 1% PFHM II, Antibiotic-Antimycotic), which is the induction medium B (induction medium b1).

### Step B: Spheroid formation and early kidney organoid formation

Step B includes inducing the early kidney organoid by: culturing the intermediate mesoderm cells formed in the step A with fibroblast growth factor induction medium B (induction medium b2) to form an intermediate mesoderm spheroid (step b1); culturing the intermediate mesoderm spheroid with the induction medium A (induction medium a2) containing a GSK3β inhibitor (step b2); and with the induction medium B (induction medium b3) containing a fibroblast growth factor (step b3).

### (Step b1)

Step b1 includes culturing the intermediate mesoderm cells formed in the step A with the induction medium b2 containing a fibroblast growth factor to form an intermediate mesoderm spheroid. The step b1 may further include detaching the intermediate mesoderm cells formed in the step A from the culture plate with a cell detachment solution. Treatment with a cell detachment solution separates intermediate mesoderm cells to provide a cell suspension. Therefore, the step b1 may further include separating the intermediate mesoderm cells formed in the step A to provide a cell suspension. "Cell detachment solution" means a solution containing enzymes that degrade cell adhesion molecules. The cell detachment solution contains, for example, trypsin, preferably recombinant trypsin (for example, TrypLE Select (Thermo Fisher Scientific Inc.)). Treatment with a cell detachment solution includes, for example, incubation at 37°C for three to 10 minutes or five to 10 minutes.

The step b1 includes culturing the cell suspension with the induction medium b2 containing a fibroblast growth factor. The treatment using the induction medium b2A may allow to form spheroids. The induction medium b2 preferably contains an ROCK inhibitor from the viewpoint of efficient spheroid formation. The culturing in the step b1 can be performed under a known cell culture condition. Known cell culture conditions may include maintaining cells at 37°C and 5% CO₂. The culture temperature is not limited to 37°C, and any temperature known in the field of cell culture can be used as appropriate. The CO₂ concentration is not limited to 5%, and any CO₂ concentration known in the cell culture field can be used as appropriate. The culturing in the step b1 with the induction medium b2 containing an ROCK inhibitor can be performed for 0.5 to two days, 0.5 days, one day, 1.5 days, preferably one day. If the induction medium b2 is substantially free of ROCK inhibitor, the culturing in the step b1 may include an additional day of culturing. The term "substantially free" in the context of the induction medium B (particularly, induction medium b2) means containing an ROCK inhibitor at a concentration of less than 100 nM, less than 50 nM, less than 10 nM, less than 5 nM, or less than 1 nM. "Substantially free" does not exclude being completely free of an ROCK inhibitor. The culturing in the step b1 forms a spheroid (also referred to as "intermediate mesoderm spheroid" in the present description) in which intermediate mesoderm cells are aggregated. The term "spheroid" herein refers to a three-dimensional cell aggregate formed in a test tube.

The term "ROCK inhibitor" herein refers to a compound that inhibits Rho kinase (Rho-associated, coiled-coil containing protein kinase: ROCK). The ROCK inhibitor may be, for example, N-(4-pyridinyl)-4β-[(R)-1-aminoethyl]cyclohexane-1α-carboxamide (Y-27632), Fasudil (HA1077), (2S)-2-methyl-1-[(4-methyl-5-isoquinolinyl)sulfonyl]hexahydro-1H-1,4-diazepine (H-1152), 4β-[(1R)-1-aminoethyl]-N-(4-pyridyl)benzene-1α carboxamide (Wf-536), N-(1H-pyrrolo[2,3-b]pyridin-4-yl)-4β-[(R)-1-aminoethyl]cyclohexane-1αcarboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), or N-(6-fluoro-1H-indazol-5-yl)-6-methyl-2-oxo-4-[4-(trifluoromethyl)phenyl]-3,4-dihydro-1H-pyridine-5-carboxamide (GSK429286A), or a combination thereof. The ROCK inhibitor may be, for example, Y-27632 singly or in combination of Y-27632 and other ROCK inhibitors. The ROCK inhibitor is preferably Y-27632. The induction medium B (particularly, induction medium b2) contains 1 to 50 µM, 2 to 25 µM, 5 to 15 µM, or 10 µM of the ROCK inhibitor. The induction medium B (particularly, induction medium b2) may contain an ROCK inhibitor other than Y-27632 at a concentration that exhibits an effect comparable to an ROCK inhibitory effect exhibited by Y-27632 at 1 to 50 µM, 2 to 25 µM, 5 to 15 µM, or 10 µM.

ROCK is a serine-threonine protein kinase. For example, ROCK phosphorylates myosin-binding subunit 1 (MYPT1) of myosin light chain phosphatase (MLCP) to suppress the enzymatic activity thereof. An "ROCK inhibitory effect" can be measured, for example, based on the amount of phosphorylation of MYPT1 by ROCK in the presence of a compound that may inhibit the phosphorylation effect. In one example, the ROCK inhibitory effect can be measured by measuring the amount of phosphorylation of MYPT1 by ROCK, measuring the amount of phosphorylation of MYPT1 by ROCK in the presence of a compound that may inhibit the phosphorylation effect, and comparing the amounts of phosphorylation. The ROCK inhibitory effect can be measured using a commercially available kit (for example, 96-well ROCK activity assay kit (Cell Biolabs, Inc., Catalog No.: STA-416) or ROCK Activity Immunoblot Kit (STA-416, Cell Biolabs, Inc., Catalog No.: STA-415)).

The induction medium b2 used in the step b1 may be the same or different in composition and/or dose from the induction medium b1 used in the step A. The induction medium b2 is preferably identical in composition and/or dose to the induction medium b1. The induction medium b2 is, for example, identical in composition and/or dose to the induction medium b1, except for containing an ROCK inhibitor.

### (Step b2)

Step b2 includes culturing the intermediate mesoderm spheroid formed in the step b1 with the induction medium A (induction medium a2) containing a GSK3β inhibitor. The culturing in the step b2 is performed, for example, in an air-liquid interface culture or in a liquid solvent. The culturing in the step b2 is preferably performed by air-liquid interface culture.

The air-liquid interface culture can be performed, for example by using a culture device provided with a culture well insert whose bottom surface is formed with a liquid-permeable membrane capable of holding cells and a culture base plate suited to receive the culture well insert and capable of holding a culture medium. Such a culture device is, for example, commercially available and may be, for example, the Cell Culture Insert Transparent PET membrane (Corning Incorporated). The culturing in the step b2 can be performed under a known cell culture condition. Known cell culture conditions may include maintaining cells at 37°C and 5% CO₂. The culture temperature is not limited to 37°C, and any temperature known in the field of cell culture can be used as appropriate. The CO₂ concentration is not limited to 5%, and any CO₂ concentration known in the cell culture field can be used as appropriate. The culturing can be performed for one to 24 hours, one to 12 hours, one to eight hours, one to five hours, two to four hours, or three hours.

The induction medium a2 used in the step b2 may have the same or different composition and/or dose as the induction medium a1 used in the step A. The induction medium a2 is preferably identical in composition and/or dose to the induction medium a1. The induction medium a2 is, for example, identical in composition and/or dose to the induction medium a1, except for a higher concentration of GSK3β.

### (Step b3)

Step b3 includes culturing the intermediate mesoderm spheroid cultured in the step b2 with the induction medium B (induction medium b3) containing a fibroblast growth factor. The culturing in the step b3 may form an early kidney organoid. The culturing in the step b3 can be performed under a known cell culture condition. Known cell culture conditions may include maintaining cells at 37°C and 5% CO₂. The culture temperature is not limited to 37°C, and any temperature known in the field of cell culture can be used as appropriate. The CO₂ concentration is not limited to 5%, and any CO₂ concentration known in the cell culture field can be used as appropriate. The culturing can be performed for four to 12 days, four to 10 days, five to eight days, six days, seven days, or eight days, preferably six days. The culturing in the step b3 is performed, for example, until the formation of nephrons including proximal tubules is confirmed in the cultured spheroid. In the step b3, the spheroid in which the formation of nephrons including proximal tubules are confirmed are also referred to as early kidney organoid.

The induction medium b3 may contain 20 to 1000 ng/mL, 60 to 500 ng/mL, or 100 to 300 ng/mL of FGF (preferably FGF9). The induction medium b3 may be the same or different in composition and/or dose from the induction medium b1 or b2. Preferably, the induction medium b3 contains the same composition and/or dose as the induction medium b1. The induction medium b3 contains, for example, the same composition and/or dose as the induction medium b2. Preferably, the induction medium b3 contains the same composition and/or dose as the induction medium b2, except for containing an ROCK inhibitor.

The step B can be performed, for example, according to the following procedure. The intermediate mesoderm cells are induced in the step A, and then the cells are detached using TrypLE Select (Thermo Fisher Scientific Inc.). The detached cells are suspended in STEMdiff APEL2 medium (200 ng/mL FGF9, 1 µg/mL Heparin, 1% PFHM II, Antibiotic-Antimycotic, 10 µM Y-27632) which is the induction medium B (induction medium b2), and the obtained cell suspension is seeded onto a PrimeSurface^{®} 96M plate (Sumitomo Bakelite Co., Ltd.) so as to provide 100,000 cells/well. The next day, the spheroid formed in the plate is placed on Cell Culture Insert of Cell Culture Insert Transparent PET membrane 6-well 0.4µm pore size (Corning Incorporated) so as to provide three to six spheroids/well. Cell Culture Insert containing the spheroids are placed in the wells of the corresponding culture plate. The induction medium A (induction medium a2), STEMdiff APEL2 medium (10 µM CHIR99021, 1% PFHM II, Antibiotic-Antimycotic) is added between the well of the culture plate and the lower surface of the transwell of the Cell Culture Insert to culture the spheroids at 37 °C and 5% CO₂ for three hours at the air-liquid interface. After three hours of the culture, the medium is replaced with STEMdiff APEL2 medium (200 ng/mL FGF9, 1 µg/mL Heparin, 1% PFHM II, Antibiotic-Antimycotic), which is the induction medium B (induction medium b3), and further culture is performed.

### Step C: Proximal tubule maturation

Step C includes culturing an early kidney organoid with a medium containing an RXR agonist and a PPAR agonist (also referred to as induction medium C). The early kidney organoid used in the step C is an early kidney organoid produced from a pluripotent stem cell by performing the steps A and B according to the present disclosure, or may be an early kidney organoid produced by a known method. Examples of known methods include: Takasato M., et al., Nat Cell Biol. 2014; 16:118-26 (incorporated herein by reference), or Takasato M., et al., Nature. 2015; 526:564-568 (incorporated herein by reference). In one embodiment, the early kidney organoid used in the step C is an early kidney organoid produced from a pluripotent stem cell by performing steps A and B according to the present disclosure.

The early kidney organoid used for culture in the step C includes early proximal tubules. Culturing the early kidney organoid in the step C may form a kidney organoid including matured proximal tubule cells. The kidney organoid including matured proximal tubule cells are characterized by the expression of markers according to the present disclosure.

The culturing in the step C can be performed under a known cell culture condition. Known cell culture conditions may include maintaining cells at 37°C and 5% CO₂. The culture temperature is not limited to 37°C, and any temperature known in the field of cell culture can be used as appropriate. The CO₂ concentration is not limited to 5%, and any CO₂ concentration known in the cell culture field can be used as appropriate. The culturing is performed for five to 20 days, six to 18 days, seven to 16 days, seven to 14 days, seven to 12 days, seven to 10 days, seven to nine days, or seven to eight days.

The kidney organoid including matured proximal tubule cells formed in the step C can be used as an active component in a regenerative medicine composition. The kidney organoid including matured proximal tubule cells can be used in the method for evaluating kidney damage caused by a test substance or the method for evaluating drug responsiveness to a test substance.

An "induction medium C" herein includes a basic medium and an additive including an RXR agonist and a PPAR agonist. The induction medium C can be prepared, for example, by adding the additive (solid or liquid) to the basic medium (liquid). For the basic medium related to induction medium C, the explanation regarding the basic medium related to the induction medium A applies as appropriate. The basic medium may contain polyunsaturated fatty acids, preferably linoleic acid and/or linolenic acid. When the basic medium contains polyunsaturated fatty acid (for example, linoleic acid and/or linolenic acid) at a concentration that does not affect the effects of the present invention, the RXR agonist and PPAR agonist, which are additives, may be added at the concentrations disclosed in the present description without consideration of the concentration of the polyunsaturated fatty acid in the basic medium. When the basic medium contains polyunsaturated fatty acid (for example, linoleic acid and/or linolenic acid) at a concentration that can affect the effects of the present invention, the RXR agonist and PPAR agonist, which are additives, may be added, taking into consideration the concentration of the polyunsaturated fatty acid in the basic medium. The concentration of the additive added to the induction medium C is appropriately determined by a person skilled in the art, taking into consideration the animal species that are provided with the cells used for culture. The induction medium C may further contain protein-free medium (for example, PFHM-II), antibiotics (for example, penicillin-streptomycin, gentamicin), or antibiotic-antimycotic mixtures (for example, Antibiotic-Antimycotic), or a combination thereof.

The term "RXR" is an abbreviation for Retinoid X Receptor and refers to a protein that is a member of the steroid/thyroid hormone superfamily of nuclear receptors. RXR forms heterodimers with nuclear receptors such as PPAR or retinoic acid receptor (RAR) and regulates transcriptional activity.

The term "RXR agonist" herein refers to a compound or composition that increases the transcriptional regulatory activity of a gene when combined with the retinoid X receptor (RXR). The RXR agonist is, for example, at least one selected from the group consisting of 9-cis retinoic acid (alitretinoin, CAS No.: 5300-03-8), AGN195204 (CAS No.: 220619-73-8), TTNPB (arotinoid acid, CAS No.: 71441-28-6), Adapalene (CAS No.: 106685-40-9), Bexarotene (CAS No.: 153559-49-0), Tazarotene (CAS No.: 118292-40-3), Tamibarotene (CAS No.: 94497-51-5), CH55 (CAS No.: 110368-33-7), and AM580 (CAS No.: 102121-60-8). The RXR agonist is, for example, 9-cis retinoic acid, or AGN195204. The RXR agonist is, for example, 9-cis retinoic acid.

Examples of the amount of an RXR agonist (for example, 9-cis retinoic acid) added to the induction medium C include 0.1 to 10 µM, 0.5 to 5 µM, 0.5 to 3 µM, 0.5 to 2 µM, or 1 µM. The induction medium C may contain an RXR agonist other than 9-cis retinoic acid at a concentration that exhibits an action comparable to an RXR agonist action exhibited by 9-cis retinoic acid at 0.1 to 10 µM, 0.5 to 5 µM, 0.5 to 3 µM, 0.5 to 2 µM, or 1 µM. Depending on the amount of the component having RXR agonist action contained in the basic medium used for the induction medium C, the amount of an RXR agonist to be added can be adjusted as appropriate.

The "RXR agonist action" can be measured based on, for example, the expression level of the luciferase gene located at downstream of the promoter region to which a heterodimer binds in which RXR forms the heterodimer with PPAR in the presence of a compound capable of activating RXR. In one example, the RXR agonist action can be measured by measuring the expression level of luciferase induced by a heterodimer of RXR and PPAR formed in the presence of 9-cis retinoic acid, measuring the expression level of luciferase induced by a heterodimer of RXR and PPAR in the presence of a compound capable of activating RXR, and by comparing the expression levels. The RXR agonist action can be measured using a commercially available assay (INDIGO Biosciences, Inc., nuclear receptor luciferase reporter assay).

A "PPAR" herein is an abbreviation for Peroxisome Proliferator-Activated Receptor and belongs to a member of the nuclear receptor superfamily. There are three subtypes of PPAR: PPAR alpha (A), PPAR gamma (G), and PPAR delta (D).

The term "PPAR agonist" herein refers to a compound or composition that activates a PPAR, such as PPARA, PPARG, or PPARD, or a combination thereof. The PPAR agonist is at least one selected from the group consisting of a PPARA agonist, a PPARG agonist, a PPARD agonist, a PPARA/G agonist, a PPARA/D agonist, a PPARG/D agonist, and a PPARA/G/D agonist. The PPAR agonist is preferably a PPARA agonist, a PPARG agonist, or a PPARD agonist. The PPAR agonist is preferably a PPARA agonist or a PPARG agonist. The PPAR agonist is more preferably a PPARA agonist.

In the present invention, the term "PPARA agonist" means, for example, a compound or composition that activates PPARA. The PPARA agonist is commercially available (for example, refer to https://www.medchemexpress.com/Targets/PPAR.html (incorporated in the present description by reference)). The PPARA agonist is, for example, at least one selected from the group consisting of: CP775146 (CAS No.: 702680-17-9), pirinic acid (WY14643, CAS No.: 50892-23-4), bezafibrate (CAS No.: 41859-67-0), fenofibrate (CAS No.: 49562-28-9), gemfibrozil (CAS No.: 25812-30-0), clofibrate (CAS No.: 637-07-0), ciprofibrate (CAS No.: 52214-84-3), pemafibrate (CAS No.: 848259-27-8), binifibrate (CAS No.: 69047-39-8), clinofibrate (CAS No.: 30299-08-2), clofibric acid (CAS No.: 882-09-7), nicofibrate (CAS No.: 31980-29-7), pirifibrate (CAS No.: 55285-45-5), plafibride (CAS No.: 63394-05-8), ronifibrate (CAS No.: 42597-57-9), theofibrate (CAS No.: 49562-28-9), tocofibrate (CAS No.: 50465-39-9), SR10171, GW6471 (CAS NO.: 880635-03-0), Fenofibrate (CAS NO.: 49562-28-9), Pirinixic acid (CAS NO.: 50892-23-4), GW7647 (CAS NO.: 265129-71-3), Icariin (CAS NO.: 489-32-7), Eupatilin (CAS NO.: 22368-21-4), Gemfibrozil (CAS NO.: 25812-30-0), Palmitelaidic acid (CAS NO.: 10030-73-6), NXT629 (CAS NO.: 1454925-59-7), Saroglitazar Magnesium (CAS NO.: 1639792-20-3), Saroglitazar (CAS NO.: 495399-09-2), Oleoylethanolamide (CAS NO.: 111-58-0), BMS-687453 (CAS NO.: 1000998-59-3), Gypenoside XLIX (CAS NO.: 94987-08-3), CUDA (CAS NO.: 479413-68-8), Ciprofibrate impurity A (CAS NO.: 1474058-89-3), CP-868388 free base (CAS NO.: 702681-67-2), AVE-8134 (CAS NO.: 304025-09-0), linoleic acid, linolenic acid, and PPARα-MO-1 (CAS NO.: 810677-36-2). The PPARA agonist is preferably CP775146, pirinic acid (WY14643), bezafibrate, fenofibrate, gemfibrozil, clofibrate, ciprofibrate, linoleic acid, linolenic acid, or a combination thereof. The PPARA agonist is more preferably CP775146 or pirinic acid (WY14643). More preferably, the PPARA agonist is CP775146.

The term "PPARG agonist" herein refers to a compound or composition that activates PPARG. The PPARG agonist is commercially available (for example, refer to https://www.medchemexpress.com/Targets/PPAR.html (incorporated herein by reference)). The PPARG agonist is, for example, at least one selected from the group consisting of: Rosiglitazone (CAS No.: 122320-73-4), Troglitazone (CAS No.: 97322-87-7), Pioglitazone (CAS No.: 111025-46-8) (may be deuterated), Efatutazone (CAS No.: 223132-37-4), ATx08-001 (CAS No.: CAS No.: 193012-35-0), OMS-405, CHS-131 (CAS No.: 315224-26-1), THR-0921 (CAS No.: 606932-81-4), SER-150-DN, KDT-501 (CAS No.: 1374259-84-3), GED-0507-34-Levo, CLC-3001, ALL-4, Rosiglitazone (CAS NO.: 122320-73-4), Rosiglitazone hydrochloride (CAS NO.: 302543-62-0), Troglitazone (CAS NO.: 97322-87-7), T0070907 (CAS NO.: 313516-66-4), 5-Aminosalicylic Acid (CAS NO.: 89-57-6), GW1929 (CAS NO.: 196808-24-9), Rosiglitazone maleate (CAS NO.: 155141-29-0), Astaxanthin (CAS NO.: 472-61-7), Magnolol (CAS NO.: 528-43-8), Glabridin (CAS NO.: 59870-68-7), Balaglitazone (CAS NO.: 199113-98-9), Mifobate (CAS NO.: 76541-72-5), Inolitazone dihydrochloride (CAS NO.: 223132-38-5), EHP-101 (CAS NO.: 1818428-24-8), Adelmidrol (CAS NO.: 1675-66-7), Oroxin A (CAS NO.: 57396-78-8), Cefminox sodium (CAS NO.: 75498-96-3), Methyl oleanonate (CAS NO.: 1721-58-0), 15-Deoxy-Δ-12,14-prostaglandin J2 (CAS NO.: 87893-55-8), GSK376501A (CAS NO.: 1010412-80-2), 4-O-Methyl honokiol (CAS NO.: 68592-15-4), Caulophyllogenin (CAS NO.: 52936-64-8), Darglitazone (CAS NO.: 141200-24-0), Angeloylgomisin H (CAS NO.: 66056-22-2), DS-6930 (CAS NO.: 1242328-82-0), Inolitazone (CAS NO.: 223132-37-4) and Arhalofenate (CAS NO.: 24136-23-0). The PPARG agonist is preferably rosiglitazone or troglitazone or a combination thereof.

The term "PPARD agonist" herein refers to a compound or composition that activates PPARD. The PPARD agonist is commercially available (refer to, for example, https://www.medchemexpress.com/Targets/PPAR.html, incorporated herein by reference). PPARD is, for example, at least one selected from the group consisting of Finadelpar (CAS No.: 515138-06-4), ASP0367, GW501516 (Endurabol), Pparδ agonist 5 (molecular formula: C₂₃H₂₁F₃N₂O₂S), MBX8025 (Seladelpar, CAS No.: 851528-79-5), GW0742 (CAS No.: 317318-84-6), L165041 (CAS No.: 79558-09-1), HPP-593 (CAS No.: 1604815-32-8), and NCP-1046. The PPARD agonist is preferably Finadelpar or ASP0367 or a combination thereof.

The term "PPARA/G agonist" herein refers to a compound or composition that activates PPARA/G. The PPARA/G agonist is commercially available. The PPARA/G agonist is, for example, at least one selected from the group consisting of Sarogritazar (CAS No.: 495399-09-2), Allegritazar (CAS No.: 475479-34-6), Muragritazar (CAS No.: 331741-94-7), Tesagritazar (CAS No.: 251565-85-2) and DSP-8658.

The term "PPARA/D agonist" herein refers to a compound or composition that activates PPARA/D. The PPARA/D agonist is commercially available. The PPARA/D agonist is, for example, one or both of ELA and T913659.

The term "PPARG/D agonist" herein refers to a compound or composition that activates PPARG/D. The PPARG/D agonist is commercially available. The PPARG/D agonist is, for example, one or both of linoleic acid (CAS No.: 60-33-3) and T3D-959 (CAS No.: 1258076-66-2).

The term "PPARA/G/D agonist" herein refers to a compound or composition that activates PPARA/G/D. The PPARA/G/D agonist is commercially available. The PPARA/G/D agonist is, for example, at least one selected from the group consisting of IVA337 (Lanifibranor, CAS No.: 927961-18-0), TTA (tetradecylthioacetic acid, CAS No.: 2921-20-2), Bavachinin (CAS No.: 19879-30-2), GW4148, GW9135, Benzafibrate (CAS No.: 41859-67-0), lobeglitazone (CAS No.: 607723-33-1), and CS038 (CAS No.: 743438-45-1).

Examples of the amount of the PPAR agonist (for example, CP775146) added to the induction medium C include 0.05 to 5µM, 0.1 to 3µM, 0.1 to 2µM, 0.2 to 1.5µM, 0.2 to 1µM, or 0.3 to 1µM. The PPAR agonist other than CP775146 may be added to the induction medium C at a concentration that exhibits an action comparable to the PPARA agonist action exhibited by CP775146 at 0.05 to 5 µM, 0.1 to 3 µM, 0.1 to 2 µM, 0.2 to 1.5 µM, 0.2 to 1 µM, or 0.3 to 1 µM. Depending on the amount of the component possessing a PPAR agonist action contained in the basic medium used for the induction medium C, the amount of the PPAR agonist added can be adjusted as appropriate. The PPAR agonist action can be measured in a manner similar to the RXR agonist action as described in the present description. In one example, the PPAR agonist action can be measured by measuring the expression level of luciferase induced by the heterodimer of RXR and PPAR formed in the presence of CP775146, measuring the expression level of luciferase induced by a heterodimer of RXR and PPAR in the presence of a compound capable of activating PPAR, and comparing these expression levels. The PPAR agonist action can be measured using a commercially available assay (INDIGO Biosciences, Inc., nuclear receptor luciferase reporter assay).

In the present invention, the molar concentration ratio of the RXR agonist and PPAR agonist to be added to the basic medium, in a case of 1 for the PPAR agonist, ranges from 0.3 to 10 for the RXR agonist, preferably from 0.5 to 5, more preferably from 0.5 to 4. When the basic medium contains one or both of the agonists, the ratio can be adjusted as appropriate, but the total amount is desired to be within the above range.

The step C can be performed, for example, according to the following procedure. The kidney organoid is cultured with the induction medium C, STEMdiff APEL2medium (0.3 µM CP775146, 1 µM 9-cis retinoic acid, 1% PFHM II, Antibiotic-Antimycotic), thereby promoting proximal tubule maturation.

The induction medium C may contain 0.1 to 30 µM, 0.5 to 25 µM, 1 to 20 µM, 2 to 18 µM, 5 to 15 µM, 7 to 12 µM, or 10 µM of a PPAR agonist (for example, pirinic acid). The step C can be performed, for example, according to the following procedure. The kidney organoid is cultured using the induction medium C, STEMdiff APEL2medium (10 µM pyrinic acid, 1 µM 9-cis retinoic acid, 1% PFHM II, Antibiotic-Antimycotic), thereby promoting proximal tubule maturation.

### [Non-human mammal comprising kidney organoid including matured proximal tubule cells, and method for producing the same]

One aspect of the present disclosure provides a non-human mammal comprising a kidney organoid in a kidney or a surrounding area thereof including matured proximal tubule cells. One aspect of the present disclosure provides a method for producing a non-human mammal comprising a kidney organoid in a kidney or a surrounding area thereof including matured proximal tubule cells.

A non-human mammal comprising a kidney organoid including matured proximal tubule cells can be produced by a method including introducing a kidney organoid including matured proximal tubule cells into a kidney or a surrounding area of a non-human mammal. The non-human mammal can be produced by a method including introducing a kidney organoid including matured proximal tubule cells into a kidney of a non-human mammal or a surrounding area thereof, and breeding the non-human mammal. Breeding of a non-human mammal includes, for example, feeding known food for a non-human mammal. From the perspective of reducing graft rejection, the non-human mammal is preferably the same species and more preferably the same individual as the animal providing a pluripotent stem cell, intermediate mesoderm cell, or early kidney organoid used to produce a kidney organoid including matured proximal tubule cells.

In one example, tumor cells or tumor fragments are introduced into a kidney organoid, and the kidney organoid is introduced into a kidney of a non-human mammal or a surrounding area thereof, thereby allowing to produce a non-human mammal comprising the kidney organoid in the kidney or the surrounding areas as a kidney cancer model. The non-human mammal as a kidney cancer model can be used in a method for evaluating the effect of a candidate drug for treating kidney cancer. From the viewpoint of reducing graft rejection, the non-human mammal as a kidney cancer model is preferably the same species and more preferably the same individual as the animal providing a pluripotent stem cell, intermediate mesoderm cell, or early kidney organoid used to produce the kidney organoid into which tumor cells or tumor fragments are introduced.

A "non-human mammal" herein may be, for example, a rodent such as a mouse, rat, guinea pig, and hamster; a non-human primate such as a chimpanzee; an artiodactyl such as a cow, goat, and sheep; a perissodactyla such as a horse; and a pet animal such as a rabbit, dog, and cat. The non-human mammal is preferably a rodent or a non-human primate.

### [Regenerative medicine composition]

One aspect of the disclosure provides a regenerative medicine composition, including a kidney organoid including matured proximal tubule cells according to the present disclosure.

A "regenerative medicine composition" herein includes a kidney organoid including matured proximal tubule cells according to the present disclosure. The regenerative medicine composition according to the present disclosure can be used to treat kidney damage or disease in mammals. The regenerative medicine composition may include, for example, a pharmaceutically acceptable carrier as appropriate. The term "pharmaceutically acceptable carrier" refers to any component that is highly safe and has low allergic reactivity in mammals, except for the kidney organoid. The pharmaceutically acceptable carrier includes, for example, an aqueous or non-aqueous solvent suitable for pharmaceutical administration; a solution (for example, physiological saline, basic medium, or cell suspension preservation solution); an antifreeze agent (for example, glycerol); a water-soluble polymer (for example, dextran); or a buffer (for example, phosphate buffer). The regenerative medicine composition can be produced as appropriate according to a known method.
In one example, the regenerative medicine composition according to the present disclosure can be produced by blending the kidney organoid and a pharmaceutically acceptable carrier (for example, basic medium).

A regenerative medicine composition is administered to a mammal that needs treatment, for example, by surgically transplanting into a specific area of the kidney, or by injecting into a specific area of the kidney with an instrument such as a syringe. From the viewpoint of reducing graft rejection, a mammal to which the regenerative medicine composition is administered is preferably the same species and more preferably the same individual as the animal providing a pluripotent stem cell, intermediate mesoderm cell, or early kidney organoid used to produce the kidney organoid including matured proximal tubule cells.

The term "mammal" relating to a regenerative medicine composition is, for example, a human or a non-human mammal. The non-human mammal may be, for example, a rodent such as a mouse, rat, guinea pig, and hamster, a non-human primate such as a chimpanzee, an artiodactyl such as a cow, goat, and sheep, a perissodactyla such as a horse, and a pet animal such as a rabbit, dog, and cat. In one embodiment, the mammal is a human.

A "kidney damage" or "kidney disease" may be, for example, a kidney damaged by trauma, a disease of the kidney (for example, atrophic kidney, renovascular hypertension, amyloid kidney, hyperuricemic nephropathy, and renal tubular acidosis), a chronic kidney disease (for example, chronic glomerulus nephritis, chronic tubulointerstitial nephritis, chronic pyelonephritis, and chronic kidney failure), a syndrome related to chronicity (for example, Gitelman syndrome, Bartter syndrome, Fanconi syndrome, Lowe syndrome, nephrotic syndrome), or kidney cancer.

The regenerative medicine composition according to the present disclosure can be used in a method for treating kidney damage or disease in mammals. One embodiment provides a method for treating kidney damage or disease, comprising administering a regenerative medicine composition including a kidney organoid including matured proximal tubule cells according to the present disclosure to a mammal that needs treatment.

### [Method for treating kidney damage or disease]

One aspect of the disclosure provides a method for treating kidney damage or disease in mammals.

A method for treating kidney damage or disease in a mammal includes introducing a kidney organoid including matured proximal tubule cells or a regenerative medicine composition into a kidney of a mammal that needs treatment or a surrounding area thereof. The kidney organoid introduced into a mammal may be, for example, in the form of an organoid or in the form of single cells and/or cell clusters composed of multiple cells. In one example, a method for treating kidney damage or disease in a mammal includes injecting a population of kidney organoid-derived cells including matured proximal tubule cells into a kidney of a mammal that needs treatment or a surrounding area thereof.

The term "mammal that needs treatment" herein means a mammal that has or is suspected of having kidney damage or disease. A mammal with kidney damage or disease refers to a mammal that has been diagnosed as having kidney damage or disease by a health care professional (for example, a physician) according to predetermined diagnostic criteria. The mammal suspected of having kidney damage or disease may be a mammal suspected of having kidney damage or disease, for example, based on the mammal's behavioral history (for example, trauma, and having received or receiving drugs harmful to kidney tissue) or medical history (for example, suffering from or having had suffered from kidney disease according to the present disclosure, chronic kidney disease, kidney-related syndrome, or kidney cancer). The mammal according to the present embodiment is, for example, a human or a non-human mammal. The non-human mammal may be, for example, a non-human primate such as a chimpanzee, an artiodactyla such as a cow, goat, or sheep, a perissodactyla such as a horse, or a pet animal such as a rabbit, dog, or cat. The mammal according to the present embodiment is preferably a human or non-human primate, more preferably a human.

"Treatment" of kidney damage or disease includes maintaining, reducing, or eliminating symptoms or pathology thereof. Treatment of kidney damage or disease includes curing kidney damage or disease.

From the viewpoint of reducing graft rejection, a mammal that needs treatment is preferably the same species or the same individual as the animal providing a pluripotent stem cell, intermediate mesoderm cell, or early kidney organoid used to produce a kidney organoid including matured proximal tubule cells or a regenerative medicine composition to be introduced into the mammal.

### [Evaluation method]

One aspect of the present disclosure provides a method for evaluating drug responsiveness to a test substance, the method including: contacting the kidney organoid including matured proximal tubule cells according to the present disclosure or a non-human mammal having the kidney organoid with a test substance; and measuring drug responsiveness in the kidney organoid or the non-human mammal to the test substance.

A "test substance" herein may be, for example, a low-molecular-weight compound, a protein (for example, an antibody), DNA, RNA, a low-molecular-weight interfering RNA, or an antisense oligonucleotide. The test substance may be, for example, drugs or candidate substances thereof for treating diseases in mammals (for example, diseases in organs such as the kidney, bladder, stomach, and intestine) or cancer (for example, kidney cancer, bladder cancer, stomach cancer, and intestinal cancer). The test substance may be, for example, a substance known or being likely to cause kidney damage. For example, the test substance may be one type, or a mixture of two or more types. The test substance is preferably one type of substance.

"Contacting" a test substance with a kidney organoid including matured proximal tubule cells or a non-human mammal including the kidney organoid means that the kidney organoid or the non-human mammal is placed in a situation where the test substance can come into contact with the kidney organoid or the non-human mammal. Contacting the kidney organoid with the test substance may be, for example, mixing the test substance with a culture solution containing the kidney organoid. Contacting the non-human mammal with the test substance may be, for example, orally or parenterally administering the test substance to the non-human mammal.

The drug responsiveness includes, for example, changes in the structural or functional properties of the kidney organoid including matured proximal tubule cells due to the test substance. In this example, measuring the drug responsiveness includes measuring structural or functional properties of the kidney organoid. The structural properties may be, for example, the proportion of matured proximal tubule cells in the kidney organoid. The functional properties may be, for example, the amount of dextran uptake in the kidney organoid. The dextran uptake by the kidney organoid can be measured, for example, according to the method described in Cell Stem Cell 25, 373-387, September 5, 2019 (incorporated in the present description by reference) or Journal of Biological Methods, 2021, Vol. 8(2)e150.

The change in structural properties includes, for example, comparing the proportion of matured proximal tubule cells in a kidney organoid including matured proximal tubule cells not in contact with a test substance (hereinafter referred to as "first proportion") and, the proportion of matured proximal tubule cells in the kidney organoid including matured proximal tubule cells that have been contacted with the test substance (hereinafter referred to as "second proportion"). The change in the structural properties may be, for example, the ratio of the second proportion to the first proportion (= [second proportion] / [first proportion]). The ratio of 0.7 indicates a 30% reduction in matured proximal tubule cells upon contact with the test substance. This reduction in matured proximal tubule cells is thought to be due to, for example, cell death such as apoptosis being induced by contact between the test substance and the cells. This reduction in matured proximal tubule cells indicates that the test substance is nephrotoxic. Therefore, the evaluation method according to the present disclosure includes evaluating the nephrotoxicity of the test substance based on the measurement results of drug responsiveness. In another example, the evaluation method according to the present disclosure includes evaluating the nephrotoxicity of the test substance to a kidney organoid including matured proximal tubule cells or to a non-human mammal having the kidney organoid based on the measurement result of drug responsiveness. Herein, examples of the test substance with nephrotoxicity include, but not limited to, antibiotics and antibacterial drugs (aminoglycosides, vancomycin, penicillin, and the like), nonsteroidal anti-inflammatory drugs (ibuprofen, indomethacin, fenoprofen, and the like), anticancer drugs (cisplatin and the like), contrast media, immunosuppressants (cyclosporine and the like).

The proportion of matured proximal tubule cells in the kidney organoid according to the present disclosure can be calculated, for example, by separating the cells constituting the kidney organoid into single cells, and assigning the number of matured proximal tubule cells in the separated cell population by the total number of cells in the separated cell population. As a method for isolating single cells from the kidney organoid including matured proximal tubule cells, a known method for separating individual cells from cell clusters (for example, spheroid or organoid) can be used. Individual cells from the kidney organoid can be obtained, for example, by performing "preparation of a kidney organoid-derived single cell" according to the present disclosure. The number of matured proximal tubule cells, the total number of cells, or the proportion of matured proximal tubule cells in the separated cell population can be determined, for example, by FACS. Measurement of matured proximal tubule cells by FACS can be performed, for example, by using lectins (for example, LTL) or antibodies that specifically bind to proximal tubules according to the present disclosure. The antibody may be labeled with a known fluorescent substance.

The change in functional properties includes, for example, comparing the amount of dextran uptake in the kidney organoid including matured proximal tubule cells that are not in contact with the test substance (hereinafter referred to as "first uptake amount"), and the amount of dextran uptake in the kidney organoid including matured proximal tubule cells that have been contacted with the test substance (hereinafter referred to as "second uptake amount"). The change in the functional properties may be, for example, the ratio of the second uptake amount to the first uptake amount (= [second uptake amount] / [first uptake amount]). The first uptake amount and the second uptake amount are preferably measured with the same area of the proximal tubule. The ratio of less than one indicates that the functional properties of the kidney organoid including matured proximal tubule cells have been reduced by contact with the test substance. Therefore, the evaluation method according to the present disclosure includes evaluating the decrease in kidney function due to the test substance based on the measurement result of drug responsiveness. In another example, the evaluation method according to the present disclosure includes evaluating the decrease in kidney function due to a test substance for the kidney organoid including matured proximal tubule cells or for a non-human mammal having the kidney organoid, based on the result of the drug responsiveness measurement.

The amount of dextran uptake in the kidney organoid including matured proximal tubule cells can be measured, for example, by culturing the kidney organoid in a medium supplemented with fluorescently labeled dextran for a predetermined period of time (for example, 24 hours), and then detecting the fluorescence emitted from the kidney organoid. The fluorescence can be measured using, for example, a fluorescence microscope.

In one example, the measurement of drug responsiveness includes: measuring structural or functional properties in a kidney organoid including matured proximal tubule cells not in contact with a test substance to obtain a first measurement result; measuring structural or functional properties in a kidney organoid including matured proximal tubule cells that has been contacted with a test substance to obtain a second measurement; and comparing the first measurement result and the second measurement result. In one example, the evaluation method according to the present disclosure includes evaluating drug responsiveness (for example, the presence or absence of kidney function or the level thereof) in a kidney organoid including matured proximal tubule cells treated with the test substance or a non-human mammal having the kidney organoid, based on the measurement result of the drug responsiveness.

The drug responsiveness may be the average value of the results measured from, for example, a kidney organoid including at least three (for example, three, four, five, six, seven, or eight or more) matured proximal tubule cells or a non-human mammal having the kidney organoid.

### [Method for promoting proximal tubule maturation]

One aspect of the present disclosure provides a method for promoting proximal tubule maturation. The method includes culturing an early kidney organoid with a medium containing an RXR agonist and a PPAR agonist. The method for promoting proximal tubule maturation is performed in vitro.

The term "proximal tubule" herein refers to the region in the kidney organoid where LTL-binding cells cluster. The proximal tubule, for example, has a brush border. In one example, if the expression level of proximal tubule marker in the kidney organoid (target organoid) obtained by culturing the early kidney organoid with a medium containing an RXR agonist and a PPAR agonist is significantly higher than the expression level of the proximal tubule marker in the kidney organoid (control organoid) obtained by culturing the early kidney organoid with a medium substantially free of either or both of an RXR agonist and PPAR agonist, proximal tubule maturation may be evaluated to be promoted. In another example, if the number of endosomes with a predetermined size or more in the target organoid is significantly higher than the number of endosomes with a predetermined size or more in the target organoid, proximal tubule maturation may be evaluated to be promoted. A predetermined size in the context of endosomes is, for example, 0.5 µm². In another example, if the amount of dextran uptake in the target organoid is significantly higher than the amount of dextran uptake in the target organoid, proximal tubule maturation may be evaluated to be promoted.

The term "comprising" herein means that the listed element and/or step is present and that other elements and/or steps may be added. The term "consisting of" herein means that the listed element and/or step is present and other elements and/or steps are excluded. The term "consisting essentially of" herein means that the listed elements and/or steps are present, and other elements and/or steps may be added as long as the novel technical features of the cell layers, organoids, compositions, and methods are not affected. The term "substantially free" herein does not exclude "completely free".

Descriptions of terms, aspects, and embodiments provided by the present disclosure apply as appropriate between corresponding terms, aspects, and embodiments provided by the present disclosure, unless otherwise specified.

Hereinafter, specific examples will be described, but represent preferred embodiments of the present invention and are not intended to limit the invention described in the appended claims in any way.

### [Examples]

### Materials and methods

In Examples 2 and 3 described below, the following growth factors and compounds were used:
CHIR99021 (Tocris Bioscience, #4423), Recombinant Human FGF9 (R&D Systems, Inc., #273-F9-025), Heparin (Sigma-Aldrich, #H4784-250MG), CP775146 (Tocris Bioscience, #4190), and 9-cis retinoic acid (Abcam plc., #ab141023).

### (Preparation of single cells derived from kidney organoid)

The kidney organoid was transferred to a centrifuge tube, and a cell dissociation solution (DPBS (+), 5 mg/mL Bacillus Licheniformis Protease (Sigma-Aldrich), 125 U/mL DNase (Roche)) was added to the centrifuge tube. Up to 12 organoids were placed in one centrifuge tube. The centrifuge tube was incubated at 6°C for 30 minutes. During the 30-minute incubation, pipetting was performed every five minutes to prepare a cell suspension containing cells separated from the organoids. To 1 mL of the obtained cell suspension, 4 mL of ice-cold diluted solution A (DPBS (-), 0.5% FBS, 2 mM EDTA, 125 U/mL DNase) was added. The centrifuge tube containing the diluted cell suspension was centrifuged at 300 rcf and 4°C for 10 minutes, and the supernatant was removed. An ice-cooled diluted solution A was added to the precipitate for resuspension. The cell suspension was centrifuged again and the precipitate was resuspended in the diluted solution A. The obtained cell suspension was transferred to a tube with a 35 µm filter (Falcon) and passed through the filter to prepare a cell suspension containing single cells.

### (Obtaining LTL-positive proximal tubular cells with MACS method)

LTL-biotin (1 : 50) was added to the prepared cell suspension containing single cells and incubated at 4°C for 15 minutes. Thereafter, the cell suspension was centrifuged at 300 rcf and 4°C for five minutes, and the supernatant was removed. An ice-cooled diluted solution A was added to the precipitate for resuspension. Centrifuging the cell suspension and obtaining a cell suspension from the precipitate were performed two more times. Anti-biotin microBeads Ultra-Pure was added to the obtained cell suspension in the diluted solution A and incubated at 4°C for 15 minutes. Thereafter, the cell suspension was centrifuged at 300 rcf and 4°C for five minutes, and the supernatant was removed. The ice-cooled diluted solution A was added to the precipitate for resuspension. This operation was repeated two more times. From the obtained cell suspension in the diluted solution A, cells to which LTL binds (also referred to as LTL-binding cells or LTL-positive cells) were obtained by a MACS method. More specifically, the cell suspension was applied to an MS column (Miltenyi Biotec B.V. & Co. KG) fixed in a magnetic field, and LTL-positive proximal tubular cells were obtained as a positive fraction.

### (Analysis of gene expression level in LTL positive cells)

Total RNA was extracted from the obtained cell suspension containing LTL-positive proximal tubular cells with a Purelink RNA micro kit (Thermofisher Scientific Inc.). Using total RNA obtained as a template, cDNA synthesis was performed using PrimeScript^{™} RTmastermix (Perfect Real Time) (Takara Bio Inc.). The synthesized cDNA was quantified by real-time PCR with TB Green^{®} Premix Ex Taq^{™} II (Tli RNase H Plus) (TaKaRa Bio Inc.). Expression level analysis was performed using this quantitative PCR.

### [Example 1] Single cell RNA sequencing of cell populations derived from kidney organoid

Example 1 was performed for the purpose of identifying factors involved in the maturation of a kidney organoid. The inventors performed single-cell RNA sequencing on an organoid produced by performing a step A and a step B described below every day of differentiation induction, and clustered the cells. The result showed that over differentiation induction for 31 days, differentiation proceeded into diverse cell populations including interstitial cell populations, muscle cell populations, neuronal cell populations, glomerular cell populations, nephron progenitor cell populations, and tubular cell populations, and there was variation in the number of days to reach each cell population. Further, the result of single-cell RNA sequencing was analyzed using a pseudo-time axis. The result has found the existence of cells that exhibited specific pseudotimes of specific cell types in the organoid that was produced for a short time, that is, the existence of cells that matured quickly, and the existence of cells of the same cell type that exhibited the same pseudo-time in the organoid that was produced for a long time, that is, the existence of cells that matured slowly. This result shows the same pseudotime for specific cell types, but indicates that comparing cell populations derived from kidney organoids produced for different days may identify genes related to the maturation of that cell type. Therefore, for proximal tubular cells, gene expression variation analysis was performed on cell populations derived from kidney organoids that show the same pseudo-time but different days of production. In this gene analysis, genes with increased expression levels and low p-values were selected from among the upstream regulatory factors.

The result showed that PPARA-related genes, PPARG-related genes, and RXR-related genes were activated. Specifically, activated PPARA-related genes includes PPARA (2.6 times, p = 2.0E-12), pirinixic acid (3.1 times, p = 4.3E-11), bezafibrate (2.6 times, p = 4.8E-9), fenofibrate (2.4 times, p = 1.4E-7), gemfibrozil (2.8 times, p = 7.2E-7), and clofibrate (2.8 times, p = 9.0E-6). In addition, for PPARG-related genes, resiglitazone (3.0 times, p = 1.2E-5) and PPARG (2.6 times, p = 1.3E-5) were activated, and for RXR-related genes, alitretinoin (2.4 times, p = 5.9E-3) and AGN194204 (2.0 times, p = 7.4E-3) were activated.

The result of Example 1 indicates that mature cells can be induced in a kidney organoid culture by using a PPARA agonist, a PPARG agonist, or an RXR agonist, or a combination thereof.

### [Example 2] Production of kidney organoid including matured proximal tubule cells

Example 2 verified an effect of promoting the maturation of a kidney organoid by a PPARA agonist, PPARG agonist, RXR agonist, or a combination thereof, as indicated by Example 1. Figure 1 is a flow chart diagram outlining the production of the kidney organoid including matured proximal tubule cells according to one embodiment. Figure 1 shows step A of inducing differentiation from a human iPS cell to intermediate mesoderm, step B of inducing differentiation from the intermediate mesoderm to an early kidney organoid, and step C of inducing differentiation from the early kidney organoid to a kidney organoid including matured proximal tubule cells. Figure 1 further shows a step of evaluating nephrotoxicity, which will be described below. In Example 2, steps A to C shown in Figure 1 were performed to produce kidney organoids including matured proximal tubule cells. In the step C of Example 2, the kidney organoid was cultured with an induction medium C containing a combination of a PPARA agonist CP775146 and an RXR agonist 9-cis retinoic acid.

### Preculture of cells for differentiation induction

Human iPS cells (1502.3 shares, transferred from Dr. Melissa Little, Murdoch Children's Research Institute) were maintained and cultured in StemFit AK02N medium (REPROCELL Inc.) on the surface of a culture plate coated with iMatrix-511 (Nippi Inc.). The cells were detached by incubating them at 37°C for five minutes using TrypLE Select (Thermo Fisher Scientific Inc.) to provide a cell suspension. The cell suspension was centrifuged at 200 rcf for five minutes at room temperature, the supernatant was removed, and the precipitate was resuspended in StemFit AK02N (10 µM Y-27632). To the cell suspension, iMatrix-511 (Nippi Inc.) was added so as to have a concentration of 0.25 µg/cm², seeded on a 12-well plate (Corning Incorporated) so as to have 15,000 cells/cm², and cultured for one day at 37°C and 5% CO₂.

### Step A: Induction of differentiation of intermediate mesoderm

Then, the culture medium used in the above-described culture was replaced with STEMdiff APEL2 medium (STEMCELL Technologies) (8 µM CHIR99021, 1% PFHM II, Antibiotic-Antimycotic), which was an induction medium A, and cultured for five days to induce a primitive streak from the iPS cells. The medium was exchanged every day from the fourth day onwards. The iPS cells clustered to form colonies on the first day of differentiation induction, and the cell colonies began to spread outward on the second day of differentiation induction. On the third day of differentiation induction, the proliferated cells began to be stacked near the center of the cell colonies, and after the fourth day of differentiation induction, the colonies took on a rounded shape. On the fifth day of differentiation induction, the induction medium A was changed to the induction medium B, STEMdiff APEL2 medium (200 ng/mL FGF9, 1 µg/mL Heparin, 1% PFHM II, Antibiotic-Antimycotic).

The cell colonies were further cultured in the induction medium B for three days to induce differentiation from the primitive streak to an intermediate mesoderm. During this culture, the shape of the colonies gradually changed from round to sheet-like.

### Step B: Spheroid formation and early kidney organoid formation

### (Step b1)

After intermediate mesoderm induction (eighth day of differentiation induction), the cells were detached by incubating them at 37°C for seven minutes with TrypLE Select (Thermo Fisher Scientific Inc.) to provide a cell suspension. The cell suspension was centrifuged at 400 rcf for three minutes at room temperature, the supernatant was removed, and the precipitate was resuspended in the induction medium B, STEMdiff APEL2 medium (200 ng/mL FGF9, 1 µg/mL Heparin, 1% PFHM II, Antibiotic-Antimycotic, 10 µM Y-27632). The cell suspension was seeded onto a PrimeSurface^{®} 96 M plate (Sumitomo Bakelite Co., Ltd.) so as to provide 100,000 cells/well. The next day (nineth day of differentiation induction), a spheroid was formed in the well.

### (Step b2)

The formed spheroids were taken out and placed on Cell Culture Insert of Cell Culture Insert Transparent PET membrane 6-well 0.4 µm pore size (Corning Incorporated) so as to provide six spheroids per well. The Cell Culture Insert containing the spheroids was placed in corresponding wells of the culture plate. STEMdiff APEL2 medium (10 µM CHIR99021, 1% PFHM II, Antibiotic-Antimycotic), which is the induction medium A, was added between the well of the culture plate and the lower surface of the transwell of the Cell Culture Insert to culture the spheroids at 37°C under 5% CO₂ for three hours at the air-liquid interface.

### (Step b3)

After three hours of the culture, the induction medium A was replaced with STEMdiff APEL2 medium (200 ng/mL FGF9, 1 µg/mL Heparin, 1% PFHM II, Antibiotic-Antimycotic), which is the induction medium B, and the spheroids were further cultured for six days (until the 15th day of differentiation induction) to induce early kidney organoids. On the 11 to 12 days of differentiation induction, formation of nephrons including proximal tubules was not observed in the spheroid. On the 15th day of differentiation induction, nephron-like structures containing early proximal tubules were observed.

### Step C: Proximal tubule maturation

On the 15th day of differentiation induction, the induction medium C was replaced with STEMdiff APEL2 medium (0.3 µM CP775146, 1 µM 9-cis retinoic acid, 1% PFHM II, Antibiotic-Antimycotic), and the obtained early kidney organoids were further cultured for eight days (until the 23rd day of differentiation induction).

In the step C, there was used the early kidney organoids in which the nephron-like structures including early proximal tubules had been observed. It is considered that if spheroids in which the formation of nephrons including proximal tubules cannot be observed are cultured with a medium containing an RXR agonist (for example, 9-cis retinoic acid), the spheroids progress in a different direction of differentiation, and the kidney organoid including matured proximal tubules cannot be efficiently produced. For example, in the step B, if intermediate mesoderm cells are cultured with an induction medium containing an RXR agonist (for example, 9-cis retinoic acid), there are formed organoids in which Wolffian ducts (derived from the anterior IM) are predominantly derived. This result forms the organoids with reduced nephron progenitor cells (derived from the posterior IM), and thus kidney organoids including matured proximal tubule cells cannot be efficiently produced.

On the 23rd day of differentiation induction, the degree of maturation of the proximal tubule was examined. The degree of maturation was determined based on the expression level of maturation markers of proximal tubular cells in the kidney organoid by the MACS method with LTL. In the group cultured in the induction medium C from the 15th day to the 23rd day of differentiation induction (CP0.3_9cisRA1 group or Mature group), the expression levels of proximal tubule marker genes were significantly increased (Figure 2a: UGT2A3, Figure 2b: AZGP1, Figure 2c: SLC39A4, Figure 2d: BHMT, Figure 2e: ACE2, Figure 2f: AQP6, Figure 2g: CUBN, Figure 2h: LRP2, and Figure 2i: ABCB1), compared to the group (Control group) cultured in STEMdiff APEL2 medium (0.2% DMSO, 1% PFHM II, Antibiotic-Antimycotic), which was a culture medium in which CP775146 and 9-cis retinoic acid were removed from the induction medium C. These results show that culturing the kidney organoid in the presence of a combination of a PPARA agonist (for example, CP775146) and an RXR agonist (for example, 9-cis retinoic acid) can induce matured proximal tubule cells in a shorter period of time than a conventional method.

In the CP0.3_9cisRA1 group, the expression level of the PPARA downstream gene group was also significantly increased (Figure 3a: APOA1, Figure 3b: APOC3, Figure 3c: CPT1A, Figure 3d: FABP1, Figure 3e: CYP27A1, and Figure 3f: ACOX1), compared to the Control group. These results show that culturing the kidney organoid in the presence of a combination of a PPARA agonist (for example, CP775146) and an RXR agonist (for example, 9-cis retinoic acid) can induce matured proximal tubule cells.

### [Example 3] Modification example of step C

In Example 3, in the step C, the kidney organoid was cultured with a medium containing a PPARA agonist CP775146 singly, an RXR agonist 9-cis retinoic acid singly, and a combination thereof, respectively. The concentration of PPARA in the agonist combination used in Example 3 (1 µM) was different from the concentration of PPARA in the agonist combination in Example 2 (0.3 µM).

In the step C, CP1 group was prepared by culturing, for eight days, the kidney organoid on the 15th day of differentiation induction in STEMdiff APEL2 medium (1 µM CP775146, 1% PFHM II, Antibiotic-Antimycotic), which is a medium obtained by removing retinoic acid from the induction medium C. There were prepared 9cisRA1 group obtained by culturing the kidney organoid on the 15th day of differentiation induction for eight days in STEMdiff APEL2 medium (1 µM 9-cis retinoic acid, 1% PFHM II, Antibiotic-Antimycotic), which was a medium obtained by removing CP775146 from the induction medium C, and CP1_9cisRA1 group (Example 3) obtained by the above culture in the induction medium C with a concentration of CP775146 of 1 µM.

In the same manner as in Example 2, the expression level of the proximal tubule marker gene group in each group was measured (Figure 4a: UGT2A3, Figure 4b: AZGP1, Figure 4c: SLC39A4, Figure 4d: BHMT, Figure 4e: ACE2, Figure 4f: AQP6, Figure 4g: CUBN, Figure 4h: LRP2, and Figure 4i: ABCB1). Compared to the Control group, in the CP1_9cisRA1 group obtained by the culture with the induction medium C, a tendency for the expression levels of many proximal tubule marker genes to increase was observed (Figure 4a: UGT2A3, Figure 4c: SLC39A4, Figure 4e: ACE2, Figure 4g: CUBN, Figure 4h: LRP2, and Figure 4i: ABCB1). No significant difference was observed in the expression levels of almost all proximal tubule marker genes among the Control group, CP1 group, and 9cisRA1 group. These results show that culturing the kidney organoid in the presence of a combination of a PPARA agonist (for example, CP775146) and an RXR agonist (for example, 9-cis retinoic acid) can induce proximal tubular cells. These results also indicate that culturing the kidney organoid in the presence of a PPARA agonist singly or RXR agonist singly fails to efficiently induce proximal tubular cells.

In the same manner as in Example 2, the expression level of the PPARA downstream gene group in each group was measured (Figure 5a: APOA1, Figure 5b: APOC3, Figure 5c: CPT1A, Figure 5d: FABP1, and Figure 5e: ACOX1). Compared to the Control group, a tendency for expression levels to increase in a large number of PPARA downstream gene groups was observed (Figure 5a: APOA1, Figure 5c: CPT1A, Figure 5d: FABP1, and Figure 5e: ACOX1). No significant difference was observed in the expression levels of almost all PPARA downstream gene groups among the Control group, CP1 group, and 9cisRA1 group. These results show that culturing the kidney organoid in the presence of a combination of a PPARA agonist (for example, CP775146) and an RXR agonist (for example, 9-cis retinoic acid) can induce matured proximal tubule cells. These results also indicate that culturing the kidney organoid in the presence of a PPARA agonist singly or an RXR agonist singly fails to efficiently induce matured proximal tubule cells.

### [Example 4] Nephrotoxicity evaluation for cisplatin

In Example 4, the steps A to C shown in Figure 1 were performed, and nephrotoxicity evaluation was also performed. Specifically, in the same manner as in Example 1, induction differentiation was performed from human iPS cells to intermediate mesoderm (step A), induction differentiation was performed from the intermediate mesoderm to early kidney organoids (step B), and further, induction differentiation was performed from the early kidney organoids into the kidney organoids including matured proximal tubule cells (step C). The kidney organoids including matured proximal tubule cells on the 23rd day of differentiation induction were subjected to the cisplatin nephrotoxicity test described below.

### Cisplatin nephrotoxicity test in kidney organoid

### (cisplatin treatment)

The kidney organoid of the Mature group (Example 2) on the 23rd day of differentiation induction was cultured for 6 days in the induction medium C without cisplatin (CDDP 0 µM) or STEMdiff APEL2 medium (cisplatin intravenous injection 10 mg "Marco" (Nichi-Iko Pharmaceutical Co., Ltd.) 5 µM, 0.3 µM CP775146, 1 µM 9-cis retinoic acid, 1% PFHM II, Antibiotic-Antimycotic) containing cisplatin. The medium was exchanged once every two days.

The Control group kidney organoid on the 23rd day of differentiation induction was cultured for six days in STEMdiff APEL2 medium (CDDP 0 µM, 1 µg/mL Heparin, 1% PFHM II, Antibiotic-Antimycotic) containing no cisplatin or in STEMdiff APEL2 medium (CDDP 5 µM, 1 µg/mL Heparin, 1% PFHM II, Antibiotic-Antimycotic) containing cisplatin. The medium was exchanged once every two days.

### (Evaluation of proportion of proximal tubular cells by flow cytometry)

A cell suspension containing single cells was prepared from a kidney organoid after cisplatin treatment according to "Preparation of single cells from kidney organoid" described above. The cells in the suspension were suspended in 4% PFA/PBS (-), and the resulting cell suspension was incubated at 37°C for 10 minutes. Thereafter, the cell suspension was centrifuged at 300 rcf and 4°C for five minutes, and the supernatant was removed. The precipitate was resuspended by adding 100 µL of DPBS (-), and further 900 µL of cold methanol was added and incubated on ice for 30 minutes. The cell number was adjusted so as to provide 10⁶ cells/100 µL with a FACS buffer (0.5% FBS, 2 mM EDTA, DPBS (-)). LTL-FITC (vector laboratory) was added to the obtained cell suspension at a concentration of 1 : 100, and the mixture was rotated and stirred at 4°C for 30 minutes. Thereafter, the cell suspension was centrifuged at 300 rcf and 4°C for five minutes, and the supernatant was removed. The precipitate was resuspended in the FACS buffer (0.5% FBS, 2 mM EDTA, DPBS (-)), and the cell suspension was passed through a cell strainer. The obtained cell suspension was analyzed using a flow cytometer (Agilent Technologies, Inc., NovoCyte).

### (Result)

In the kidney organoid of the Control group cultured in STEMdiff APEL2 medium (CDDP 0 µM) containing no cisplatin, the cell population strongly stained with LTL was 26.5% of the total cells (Figure 6a). In the kidney organoid of the Mature group cultured in the induction medium C containing no cisplatin (CDDP 0 µM), the cell population highly expressing LTL was 28.1% of the total cells (Figure 6c). These results show that the proportion of cells strongly stained by LTL induced in the kidney organoid in the Control group is almost equal to the proportion of cells strongly stained by LTL induced in the kidney organoid in the Mature group. As shown in Examples 2 and 3, matured proximal tubule cells were induced in the kidney organoid cultured with the induction medium C. Considering the results in Examples 2 and 3 together, the cell population strongly stained with LTL shown in Figure 6c is estimated to contain a high proportion of matured proximal tubule cells compared to the cell population strongly stained with LTL shown in Figure 6a.

In the Control group kidney organoid cultured in STEMdiff APEL2 medium containing cisplatin (CDDP 5 µM), 22.5% of cells were strongly stained with LTL (Figure 6b). Considering the percentage 26.5% of the cell population strongly stained with LTL shown in Figure 6a together, Example 4 shows that in the cell population strongly stained with LTL induced in the Control group kidney organoid, approximately 15% (= (1 - 22.5/26.5) × 100) of the cells were sensitive to cisplatin.

In the kidney organoid of the Mature group cultured in the induction medium C containing cisplatin (CDDP 5 µM), 13.6% of cells were strongly stained with LTL (Figure 6d). Considering the percentage 28.1% of the cell population strongly stained with LTL shown in Figure 6c together, Example 4 shows that in the cell population strongly stained with LTL induced in Mature group kidney organoid, approximately 52% (= (1 - 13.6/28.1) × 100) of the cells were sensitive to cisplatin.

Example 4 showed that approximately 15% of cells in the Control group were sensitive to cisplatin, compared to approximately 52% of the cells in the Mature group. This result shows that the Mature group kidney organoids of Examples 2 and 3 are more sensitive to low concentrations of cisplatin (for example, 5 µM) compared to the Control group kidney organoid. Cisplatin is known to cause kidney damage. It is known that kidney damage caused by cisplatin is mainly caused by damage to the proximal tubule. Considering these, it is indicated that the kidney organoid including matured proximal tubule cells provided by the present disclosure can detect cisplatin-induced kidney damage with high sensitivity. In addition, it is indicated that the kidney organoid including matured proximal tubule cells provided by the present disclosure can provide a method for evaluating drug-induced kidney damage that may occur in the proximal tubule.

### (Additional test)

An additional cisplatin nephrotoxicity test in the kidney organoid was performed (n = 5).

The Control group kidney organoid cultured in the presence of cisplatin (CDDP 5 µM) exhibited 18.1% of cells that were strongly stained with LTL, whereas the Mature group kidney organoid cultured in the presence of cisplatin exhibited 15.4%. A similar test was performed five times. The results showed that for the sensitivity of proximal tubular cells in the kidney organoid to low concentrations of cisplatin in the Mature group kidney organoid, the sensitivity to low concentrations of cisplatin (for example, 5 µM) was significantly higher, compared to the Control group kidney organoid (Figure 7).

### [Example 5] Promotion of maturation in proximal tubular cells

A kidney organoid was produced according to the method for producing the kidney organoid as described in Example 2. The kidney organoid on the 23rd day of differentiation induction was immunostained and photographed (Figure 8a).

### Immunostaining and clearing (IF staining and clearing)

A kidney organoid was treated with 4% paraformaldehyde for 30 minutes at 4°C, and then mixed with 0.5x CUBIC-L (1 M NaCl : CUBIC-L = 1 : 1) (Tokyo Kasei Kogyo Co., Ltd., T3740) and incubated overnight at room temperature. Then, 0.5% PBTX (0.5% TritonX100 in DPBS (-)) was added to the kidney organoid and it was incubated for 15 minutes to perform membrane permeabilization. The kidney organoid was washed three times with DPBS (-). The kidney organoid was treated with a blocking buffer (10% donkey serum + 0.3% Triton X in DPBS (-)) for three hours at room temperature, then mixed with a primary antibody and incubated overnight at 4°C. Then, after washing five times with 0.3% PBTX, a secondary antibody was added and incubated overnight at 4°C. After washing three times with DPBS (-), incubation was performed with 4% PFA at 4°C overnight, and then washing was performed three times with DPBS (-). After further adding 0.5x CUBIC-R (Tokyo Kasei Kogyo Co., Ltd., T3983) (5 mg/mL DAPI 1 : 1000) and treating at room temperature for 15 minutes, the liquid was replaced with CUBIC-R, and images were taken with a confocal laser microscope (LSM800, Carl Zeiss AG).

For the staining pattern of LTL that binds to the proximal tubule marker, the expression pattern of the distal tubule marker ECAD, and the expression pattern of the glomerulus marker NPHS1, no differences were observed between the Control kidney organoid (Figure 8a, top row) and the Mature kidney organoid (Figure 8a, bottom row). Cells constituting the kidney organoid on the 29th day of differentiation induction were subjected to a flow cytometer, and the proportion of proximal tubular cells (PT cells) in all cells was measured (Figure 8b). The proportion of proximal tubular cells in the Control kidney organoid was 26.5%, and the proportion of proximal tubular cells in the Mature kidney organoid was 28.1%. A similar analysis was performed five times. As a result, there was no significant difference between the proportion of proximal tubular cells in the Control kidney organoid and the proportion of proximal tubular cells in the Mature kidney organoid (Figure 8c, unpaired t-test).

Example 5 shows that the increased expression level of maturation marker genes in the kidney organoid of the Mature group shown in Examples 2 and 3 was not a result of promoted differentiation of nephron progenitor cells into proximal tubular cells, but rather a result of promoted maturation of proximal tubular cells. The examples in the present description show that a combination of PPARA and RXR agonists promotes the maturation of proximal tubular cells in the production of the kidney organoid.

### [Example 6] Expression levels of LRP2 and CUBN involved in protein reabsorption

The function of the proximal tubule includes protein reabsorption. For example, albumin (a protein) in raw urine is taken up by endosomes in the proximal tubule. The albumin taken up into endosomes is either released into blood vessels and recycled, or decomposed into an amino acid in lysosomes, and the amino acid is recycled. LRP2 and CUBN are involved in protein reabsorption.

In the same manner as in Example 2, the kidney organoid for the Control group and the kidney organoid for the Mature group were produced. The expression levels of LRP2 and CUBN in the organoid were measured on the 20th, 23rd, 26th, and 29th days of culture (Figures 9a and 9b). The expression levels of LRP2 and CUBN were each normalized to the expression level of β-actin (ACTB). The expression increased in the kidney organoid of the Mature group on the 26th day of differentiation induction, compared to the kidney organoid of the Control group (Figures 9a and 9b). Similar results were obtained for UGT2A3, AZGP1, SLC39A4, BHMT, ACE2, AQP6, and ABCB1. These results show that the kidney organoid in the Mature group matures earlier than the kidney organoid in the Control group for protein reabsorption in the proximal tubule.

### [Example 7] Number of endosomes in kidney organoid

### (Quantification of endosome number by TEM)

A tissue sample for photographing with a transmission electron microscope (TEM) was prepared as follows. The kidney organoid on the 26th day of culture was fixed using phosphate buffered 2% glutaraldehyde, and then maintained using 2% osmium tetroxide in an ice bath for three hours for fixation. The fixed organoid was dehydrated using an ethanol solution and embedded in epoxy resin. Sections of the embedded organoid were prepared using an ultramicrotome method. The sections were stained with uranyl acetate for 10 minutes, followed by lead staining solution for five minutes. The stained sections were subjected to TEM observation (Hitachi, Ltd., H-7600 at 100 kV). The acquired images were used to measure and quantify the cytoplasmic area (µm²) of proximal tubular cells and the number and size (µm²) of endosomes by using Image J Fiji.

### (Result)

The number of endosomes per cytoplasm (µm²) was significantly higher in the kidney organoid from the Mature group than in the kidney organoid from the Control group (Figure 10a). The kidney organoid cells with 0% of the proportion of endosomes larger than 0.5 µm² detected in one cell exhibited 65.4% for the Control group kidney organoid, whereas exhibited as low as 38.5% for the Mature group kidney organoid (Figures 10b and 10c). Cells with 1 to 20% of the proportion of endosomes larger than 0.5 µm² exhibited 15.4% in the Control group kidney organoid, whereas exhibited as high as 34.6% in the Mature group kidney organoid (Figures 10b and 10c). Cells with 41 to 60% of the proportion of endosomes larger than 0.5 µm² exhibited 3.8% in the Control group kidney organoid, whereas exhibited as high as 7.7% in the Mature group kidney organoid (Figures 10b and 10c). These results show that for the kidney organoid of the Mature group, the number of endosomes is significantly higher than that in the cytoplasmic region, and the proportion of endosomes larger than 0.5 µm² is higher, compared to the Control group kidney organoid.

In a fetal rat, as development progresses, Small Vacuoles and Large Vacuoles increase in the proximal tubular region (Okada T, et al., Ann Anat. 1993 Feb; 175(1): 89-94, Journal of Ultrastructure Research 53, 254-270 (1975)). In the above literature, Small Vacuoles refers to vacuoles with a diameter of less than 0.5 µm. Therefore, Example 7 shows that the kidney organoid in the Mature group contains more mature cells than the kidney organoid in the Control group.

### [Example 8] Dextran uptake by kidney organoid

### Dextran uptake assay

pHrodo^{™} Red Dextran (10,000 MW) for Endocytosis (Invitrogen Corporation, P10361) was added to the medium containing the 26th-day kidney organoid cultured in the same manner as in Example 2 so as to provide a concentration of 10 µg/mL, and the mixture was incubated at 37°C, and cultured for 24 hours under 5% CO₂. The kidney organoid was then washed three times with ice-cold DPBS (-) and maintained in DPBS (-) (1 : 100) containing LTL-FITC (Vector Laboratories, Inc., FL-1321) for two hours at 4 °C, and then, the proximal tubules were stained with LTL. A 2×2 tile image of the stained kidney organoid was acquired using CV8000 (Yokokawa, X20 lens). Fluorescent images were acquired every 5 µm over 100 µm in the Z-axis direction (thickness). The CV8000 can emit light at 488 nm and 561 nm and was equipped with BP525/50 and BP600/37 filters. The LTL two-dimensional image was created by using Average Image Projection (AveIP), and the Dextran two-dimensional image was created by using Sum Image Projection (SumIP), from the three-dimensional image. Using the created images, the fluorescence intensity of Dextran within the LTL binding region was measured and quantified.

### (Result)

The fluorescence intensity of dextran was significantly stronger in the Mature group kidney organoids than in the Control group kidney organoids (Figure 11, **p < 0.01, unpaired t-test). This result shows that the kidney organoid in the Mature group have an improved function to take in extracellular substances compared to the kidney organoid in the Control group. Example 8 shows that the Mature group kidney organoid includes matured proximal tubule cells.

### [Comparative Example 1] Expression level of maturation marker of proximal tubular cells in the presence of PPARA antagonist

In the step C of Example 2 (15th day of differentiation induction), the kidney organoid was produced according to the same method as the kidney organoid production method described in Example 2, except that PPARA antagonist GW6471 was used instead of the PPARA agonist and RXR agonist. The expression level of proximal tubule marker genes in the produced kidney organoid was measured. In the step C (15th day of differentiation induction), a group cultured in STEMdiff APEL2 medium (0.2% DMSO, 1% PFHM II, Antibiotic-Antimycotic) without adding GW6471 was used as a control.

### (Result)

Using GW6471 at a predetermined concentration significantly decreased the expression levels of maturation markers of proximal tubular cells, including UGT2A3 (Figure 12a), AZGP1 (Figure 12b), BHMT (Figure 12d), ACE2 (Figure 12e), AQP6 (Figure 12f), CUBN (Figure 12g), LRP2 (Figure 12h), ABCB1 (Figure 12i), APOA1 (Figure 12j), APOC3 (Figure 12k), and FABP1 (Figure 12n). A tendency for the expression levels of SLC39A4 (Figure 12c) and CYP27A1 (Figure 121) to decrease was observed.

These results show that the PPARA antagonist may prevent early induction of differentiation into matured proximal tubule cells.

### [Comparative Example 2] Expression level of maturation marker of proximal tubular cells in presence of RXR antagonist

In the step C of Example 2 (15th day of differentiation induction), the kidney organoid was produced according to substantially the same method as the method for producing a kidney organoid as described in Example 2, except that RXR antagonist HX531 was used instead of the PPARA agonist and RXR agonist. The expression levels of proximal tubule marker genes in the produced kidney organoid were measured. In the step C (15th day of differentiation induction), a group cultured in STEMdiff APEL2 medium (0.2% DMSO, 1% PFHM II, Antibiotic-Antimycotic) without adding HX531 was used as a control.

### (Result)

Using GW6471 at a predetermined concentration significantly decreased the expression levels of maturation markers of proximal tubular cells, including UGT2A3 (Figure 13a), AZGP1 (Figure 13b), SLC39A4 (Figure 13c), BHMT (Figure 13d), ACE2 (Figure 13e), AQP6 (Figure 13f), CUBN (Figure 13g), LRP2 (Figure 13h), ABCB1 ( Figure 13i), APOA1 (Figure 13j), APOC3 (Figure 13k), CYP27A1 (Figure 131), and FABP1 (Figure 13n).

These results show that the RXR antagonist may prevent early induction of differentiation into matured proximal tubule cells.

As described above, the results of Comparative Examples 1 and 2 show that the PPARA antagonist or RXR antagonist may prevent early induction of differentiation into matured proximal tubule cells. These results confirm that the combination of a PPARA agonist and RXR agonist promotes the induction of differentiation into matured proximal tubule cells in the kidney organoid.

### [Example 9] Production of kidney organoid in presence of pirinic acid (WY14643)

In the step C of Example 2 (15th day of differentiation induction), the kidney organoid was produced according to substantially the same method as the method for producing a kidney organoid as described in Example 2, except that a PPAPA agonist (pirinic acid) was used instead of the combination of a PPARA agonist (CP775146) and an RXR agonist (9cis retinoic acid). The expression levels of proximal tubule marker genes in the prepared kidney organoids were measured (n = 3). Figure 14 shows that the PPAPA agonist pirinic acid alone increased the expression of proximal tubule markers.

Therefore, combining a PPAPA agonist (pirinic acid) with an RXR agonist is expected to promote the maturation of proximal tubular cells in the production of the kidney organoid.

## Claims

1. A method for producing a kidney organoid, comprising:
culturing an early kidney organoid with a medium containing an RXR agonist and a PPAR agonist, wherein the kidney organoid includes matured proximal tubule cells.

2. The method according to claim 1, further comprising deriving the early kidney organoid from intermediate mesoderm cells,
wherein the deriving of the early kidney organoid comprises:
culturing the intermediate mesoderm cells with an induction medium B containing a fibroblast growth factor and substantially free of an RXR agonist to form an intermediate mesoderm spheroid; and
culturing the intermediate mesoderm spheroid with an induction medium A containing a GSK3β inhibitor, then with an induction medium B containing a fibroblast growth factor and substantially free of an RXR agonist.

3. The method according to claim 2, further comprising
deriving the intermediate mesoderm cells from a pluripotent stem cell,
wherein the deriving of the intermediate mesoderm cells comprises culturing the pluripotent stem cell with an induction medium A containing a GSK3β inhibitor, then with an induction medium B containing a fibroblast growth factor and substantially free of an RXR agonist.

4. The method according to any one of claims 1 to 3, wherein the kidney organoid expresses a proximal tubule marker,
wherein the proximal tubule marker is at least one selected from the group consisting of UGT2A3, AZGP1, SLC39A4, BHMT, ACE2, AQP6, CUBN, LRP2, ABCB1, SERPINA1, APOA1, and SPARCL1.

5. A kidney organoid including matured proximal tubule cells produced by the method according to any one of claims 1 to 4.

6. A kidney organoid, wherein the kidney organoid includes matured proximal tubule cells, the kidney organoid expresses a proximal tubule marker, and the proximal tubule marker is at least one selected from the group consisting of UGT2A3, AZGP1, SLC39A4, BHMT, ACE2, AQP6, CUBN, LRP2, ABCB1, SERPINA1, APOA1, and SPARCL1.

7. A method for producing a non-human mammal comprising a kidney organoid in a kidney or a surrounding area thereof, the method comprising introducing a kidney organoid produced by the method according to any one of claims 1 to 4, or the kidney organoid according to claim 5 or 6 into a kidney of a non-human mammal or a surrounding area thereof, wherein the kidney organoid includes matured proximal tubule cells.

8. A non-human mammal comprising a kidney organoid produced by the method according to any one of claims 1 to 4, or the kidney organoid according to claim 5 or 6 in a kidney or a surrounding area thereof.

9. A regenerative medicine composition for treating kidney damage or disease, comprising a kidney organoid produced by the method according to any one of claims 1 to 4, or the kidney organoid according to claim 5 or 6.

10. A method for evaluating drug responsiveness to a test substance, comprising:
contacting a kidney organoid produced by the method according to any one of claims 1 to 4, the kidney organoid according to claim 5 or 6, a non-human mammal produced by the method according to claim 7, or the non-human mammal according to claim 8 with the test substance; and
measuring drug responsiveness in the kidney organoid or the non-human mammal to the test substance.

11. A method for promoting the maturation of proximal tubule, wherein an early kidney organoid is cultured with a medium containing an RXR agonist and a PPAR agonist.
